# EUROPEAN PATENT SPECIFICATION

(11) **EP 4 271 318 B1**
(45) Date of publication and mention of the grant of the patent: **02.10.2024**
(21) Application number: 21839646.3
(22) Date of filing: 22.12.2021
(51) Int. Cl.: A61B 46/10, A61B 90/57, A61B 90/50, A61B 90/00, A61B 46/00

(54) **APPARATUS AND METHOD OF USE FOR ENDOSCOPIC CONDUIT HARVESTING**
VORRICHTUNG UND VERFAHREN ZUR VERWENDUNG FÜR ENDOSKOPISCHE LEITUNGSENTNAHME
APPAREIL ET PROCÉDÉ D'UTILISATION POUR LA COLLECTE DE CONDUIT ENDOSCOPIQUE

(30) Priority: 30.12.2020 GB 202020742
(43) Date of publication of application: 08.11.2023
(73) Proprietor: CardioPrecision Limited, Glasgow G1 3NQ (GB)
(72) Inventor: SUTHERLAND, Fraser William Havern, Glasgow G1 3NQ (GB); SUTHERLAND, Ying, Glasgow G1 3NQ (GB); TARGELL, David John, North Ayrshire KA1 5PD (GB); DONNAN, Jeremy Francis, Edinburgh EH14 7JJ (GB)
(74) Representative: Murgitroyd & Company
(86) International application number: PCT/GB2021/053416
(87) International publication number: WO 2022/144539

(56) References cited:
- EP-A1- 1 239 805
- EP-B1- 1 239 805
- US-A- 5 571 072
- US-A- 5 732 712
- US-A1- 2015 157 188
- US-B2- 6 973 688
- US-B2- 7 074 180

## Description

### Background

This invention relates to an apparatus and method in the field of coronary artery bypass grafting (CABG) surgery. CABG is one of the commonest surgical operations performed worldwide, and in the USA alone there are over 200,000 isolated CABG procedures performed each year.

Although percutaneous coronary intervention (PCI) is a seemingly less invasive means of treating coronary artery disease, randomised controlled trials (RCTs) show the benefits of CABG over PCI in large numbers of patients with multi-vessel coronary artery disease. Patients with three vessel coronary artery disease, left main stem disease and patients with diabetes are particularly best treated by CABG.

In CABG surgery conduits, which can be either arteries or veins, are used to bypass diseased segments of coronary arteries in order to restore flow to the distal vessels during rest and exercise.

Surgeons commonly use the left internal thoracic artery (LITA) to bypass the left anterior descending artery. During harvest of the left internal thoracic artery the proximal end of the conduit is left attached at its origin and only the distal end divided and then anastomosed to the left anterior descending artery beyond the stenosed or occlusive segments. Conduits used in this way are said to be `pedicled grafts'.

The right internal thoracic artery (RITA) may also be used as a pedicled graft. Alternatively, it may be detached and used as a 'free graft', i.e. a graft transplanted without its normal attachments, or a pedicle, from one site to another. When used as a 'free graft', the proximal end needs to be anastomosed to the aorta or to another artery to provide arterial inflow, either before or after anastomosis of the distal end of the conduit to the coronary artery beyond the diseased segment or segments.

The gastro-epiploic artery (GEA) normally supplies blood to the greater curvature of the stomach. The branches of the GEA may be divided, and the distal end of the artery may be brought into close proximity of the heart through a fenestration created in the diaphragm and used as a pedicled graft during CABG. Alternatively, this conduit may be detached at both ends, removed from the abdomen and used as a free graft.

For other bypass conduits, harvested from areas of the body remote from the chest, the conduit has to be used as a free graft. Commonly used free graft conduits for CABG include the long saphenous vein, the short saphenous vein and the radial artery. Because veins typically contain valves that restrict the direction of flow, when using a venous conduit it must be reversed, so that the distal end is anastomosed onto the aorta or other inflow artery, and the proximal end is anastomosed to the coronary artery.

The most common graft configuration for a CABG operation makes use of the left internal thoracic artery harvested as a pedicle from the chest wall for revascularisation of the left anterior descending artery and reversed long saphenous vein grafts for other coronary arteries. In current practice, it is estimated that almost 80% of all bypass conduits are saphenous veins because of their ease of harvesting and the lesser technical challenge of performing multiple vein graft anastomoses as compared with performing multiple arterial graft anastomoses.

The radial artery is also fairly commonly used as a conduit for CABG. It is technically easier to perform multiple arterial anastomoses using the radial artery than to do the same grafts using both internal thoracic arteries. It is also believed by some practitioners in the field that the radial artery offers superior long term graft patency and improved patient survival compared to saphenous vein grafts. However, the technical procedure of harvesting the radial artery has historically been considered to be more challenging than that required to harvest the long saphenous vein.

Traditional harvest techniques involve open incisions in the chest, abdomen, arm or leg. Some of these incisions, such as the incision in the leg to harvest the long saphenous vein or the incision the arm to harvest the radial artery, are some of the longest incisions in surgery. The chest incision and abdominal incision for harvesting internal thoracic arteries and gastro-epiploic arteries respectively are also very invasive incisions as they involve entry into a body cavity which adds risk and some extension to the recovery time from the surgical procedure.

Therefore, there is a need for less invasive endoscopic techniques for conduit harvest that make use of much smaller incisions. Smaller incisions generally provide a superior cosmetic result, are associated with less blood and heat loss during surgery and cause less trauma. In general, they heal more quickly, with less pain, and contribute to much faster recovery, allowing patients to return to their normal daily activities and occupation more quickly.

Disposable endoscopic vessel harvesting (EVH) tools are known, such as Vasoview Hemopro 2 by Getinge and Virtuosaph Plus by Terumo Cardiovascular.

Such EVH tools and endoscopes associated with them are generally compatible with commercially available full service imaging stacks. A full service imaging stack will generally comprise a very substantial operating theatre cart equipped with a camera, advanced CO₂ insufflator, advanced light source, large screen monitor mounted on a very substantial monitor arm, gas bottles and holders, tube set adaptors, printer and connection hub to integrate all of the components.

The monitor will typically measure at least 660 mm across the screen, and may be much larger, as it is mounted at a distance from the operator and away from the immediate vicinity of the operating table. In the field of operating theatre surgical monitors, the perception of practitioners in the field is that larger is always better.

However, a full service imaging stack is very expensive and takes up precious space in operating theatres that are setup and equipped to perform CABG surgery. The stacks have to be positioned in the operating theatre, generally after the patient's skin has been prepared with antiseptic and relevant parts of the body draped, by circulating operating theatre staff. The position of the large monitor screen may need to be adjusted at multiple additional times during the procedure.

Firstly, equipping every operating theatre where CABG surgery is routinely performed with a full service imaging stack for endoscopic vein harvesting alone is considered cost prohibitive as most hospitals have no clear mechanism for recovering this cost.

Moreover, such imaging stacks occupy precious space in the operating theatre and cannot be perfectly located. Space around the operating table is especially constrained because of the competing needs of anaesthetic personnel, the anaesthetic machine, the transoesophageal echocardiography machine, the scrub table and scrub nurse, the main operating surgeon and his first surgical assistant, the cardiopulmonary bypass (CPB) machine and associated heater cooler unit and the perfusionist. Relocation of some of these elements is not always possible, because of the fixed gas and power supplies of the machines, or the fixing of the machines themselves. Their movement would require major changes to the operating theatre infrastructure. Most operating theatres have a standard configuration which is determined in part by plumbing for power and gas lines, so there is little flexibility in where these machines are placed and in the overall setup in theatres.

Because the large monitor is on a separate stack, the position of the stack and/or the monitor itself must be adjusted before or during the operative procedure by separate circulating operating theatre personnel, who might be engaged with other tasks. As time is of the essence in surgery, especially for conduit harvesting which is generally performed from different parts of the body at the same time by different surgeons or theatre practitioners, time taken for adjustment of the monitor position is detrimental.

For ease of use, ease of training, and comfort of the user throughout the surgical procedure, the screen should optimally be positioned directly opposite the EVH operator. From the foregoing, it will be understood that it is generally not possible to optimally locate a full service imaging stack immediately opposite the EVH operator for optimal viewing of the monitor screen, as this space is either occupied by the scrub nurse and scrub table or else by the cardiopulmonary bypass machine and heater cooler unit associated with it, or else by other equipment and/or key personnel. For this reason, the imaging stack must usually be located at the foot of the patient. In this position the EVH operator has to look away from the operative field whilst operating. This is known to impede dexterity, make learning new procedures more time consuming and difficult, and increase the risk of complications. Over the longer term, the unnatural position of the monitor may adversely affect operator comfort through repetitive strain injury.

The large full service stacks are also heavy and difficult for nurses to move around the operating theatre or between operating theatres. The large screens provided on such stacks have to be moved and carefully positioned by circulating operating theatre staff time before the EVH operator can begin the procedure and may need to be moved again during the procedure as the EVH operator moves one or other extremity of the legs. All of these adjustments have to be communicated to circulating operating theatre staff, the EVH operator being dependent therefore on the actions of these personnel who may be otherwise engaged in attending to the main surgeon and/or the main scrub nurse's needs or have other additional, competing demands on their time.

The image settings of the large surgical monitor may need to be adjusted by circulating operating theatre staff who may not be familiar with the operation of surgical monitor settings if the theatre is predominantly doing CABG procedures where experience with endoscopic technology may not be a core skill of personnel generally working in that theatre. It will thus be understood that it may be challenging and/or time consuming for the EVH operator to communicate image setting adjustment requirements to circulating operating theatre staff prior to commencing the procedure or during the procedure, especially if such staff are unfamiliar with their adjustment.

The resolution and quality of the captured image on a large screen has to be very good because poor image quality is magnified and may appear pixelated on the large screen making it difficult for the EVH operator to see the operative field and work effectively, and without making mistakes brought on by poor image on the large screen.

An example of a proposal to provide improved visualisation of endoscopic images is found in US 2015/0157188 which describes a video imaging subsystem, an endoscope, an optical system, an image display device coupled to a moveable support assembly which may be fixed to an edge of an operating table. The support assembly includes an upright post and a laterally extending limb for supporting a an image display device. The disclosed support assembly is designed to be easily moveable and includes one or more linkages, ball and socket joints, moveable joints and/or articulating arm or arms, or a gooseneck (a manipulatable, bendable neck that retains its position). A cover that is autoclaveable or disposable may be provided for the support. The supported screen is removable, and also can be covered by a cover that is autoclaveable or disposable. A user can operate a screw, one or more of several levers, fixing handles, swivel joints and, when present, the gooseneck to set a desired position for the screen over a selected position of the operating table. Mechanically assisted movement of the screen may be accomplished *inter alia* using an actuator, a strut and crank, cables, or a piston and spring configured to provide screen movement. It remains of interest to provide a simpler system which is easily hand adjusted directly and quickly by a user during a surgical procedure simply by touch control using one hand, so as to minimise delays and interruption during the surgical procedure and without compromising the sterile field.

### Object of the invention

What is needed is a means of bringing the screen into the operative field, orienting above the vessel harvesting incision or incisions and having its operable by the EVH operator independent of other circulating operating theatre personnel.

It is therefore an aim of the present invention to allow the user to stand in a natural, upright posture throughout the surgical procedure, or to make it easier to adjust the screen position resulting in a quicker process, or to otherwise overcome the above disadvantages of the prior art.

### Summary of the Invention

The invention is defined in appended independent claim 1.

Further embodiments are defined in appended dependent claims.

According to a **first aspect** of the present invention there is provided a monitor mount apparatus for use in an operating theatre comprising:
a mount attachable to an operating table,
a support arm connected by a clamp assembly to the mount, the support arm extending along its longitudinal axis in a horizontal plane parallel to a plane of the operating table, and
a monitor mounting bracket connected by a friction assembly to a distal end of the support arm distal from the clamp assembly, and
a display monitor adapted to display an image from an endoscope and fixedly supported by the monitor mounting bracket,
wherein the friction assembly is adapted to permit under hand operation of a user:
   tilting of the monitor mounting bracket about a tilt axis parallel to the longitudinal axis of the support arm, and
   vertical movement of the monitor mounting bracket relative to the support arm; and
wherein the friction assembly prevents said tilting and vertical movement under self weight of the display monitor and the monitor mounting bracket.

The friction assembly may further be adapted to permit, under hand operation of a user, translational movement of the monitor mounting bracket along the tilt axis.

Typically the friction assembly is adapted to permit said tilting, translational movement and vertical movement under an operating force of less than 50 N applied by the hand of a user, more preferably less than 30 N, most preferably less than 20 N. Typically the force required to move the display monitor is not more than the force which can be applied by one hand of a user. Typically the friction assembly means that the monitor comes to rest as soon as the force is reduced to beneath a threshold figure, which may be less than 5 N, or of the order of 1 N or 2 N. This means that the display monitor will not move when subject to a slight accidental contact force.

The support arm may be pivotally connected by the clamp assembly to the mount, and the clamp assembly may be operable between an unclamped state in which the support arm can rotate about a vertical axis and a clamped state in which the support arm is fixed relative to the mount in a working position.

The clamp assembly may comprise a friction brake. The clamp assembly may be a collet clamp assembly having a rotatable adjusting sleeve, which can be grasped and rotated by an operator, to adjust the frictional force of the friction brake. The clamp assembly may be operable to a frictionally clamped state in which the support arm is held in a required position by the clamp assembly when no force is applied by a user to the support arm, monitor mounting bracket connected or display monitor, and in which the support arm can be moved under hand operation of a user when sufficient force is applied by the user to the support arm, monitor mounting bracket connected or display monitor.

Typically the display monitor has a visible display area. Typically the visible display area has a maximum diagonal dimension of less than 410 mm. This has the advantage that the screen size is small enough to be in the operative field of view of the user, and the line of sight is independent of other theatre personnel.

Typically, the display monitor weighs less than 2.5 kg, more preferably less than 1.5 kg and most preferably less than 1 kg. This has the advantage that the friction assembly is capable of holding the display monitor in position against its self weight, while still allowing movement of the display monitor by one hand of a user. It is envisaged that as monitors are developed to be thinner and lighter, the display monitor of the present invention could have a larger screen with a larger maximum diagonal dimension, but still weigh less than 2.5 kg, or even less than 1 kg.

The display monitor may be a high definition monitor. Paradoxically the smaller screen size means that the quality of the captured image is less important. Older image capture systems which produce fewer pixels in total can be used effectively, without significantly affecting the quality of the image on the small screen, since a smaller screen has fewer pixels in total than a larger screen, even at the same resolution (pixels per inch).

As well as the ability of the user to adjust the position oneself, one of the main advantages of this system is that the monitor may be positioned directly in the line of sight of the operator, which makes for improved dexterity and much easier conduit harvest.

The monitor is small and may therefore be brought close to and directly opposite the EVH operator without interfering with the scrub nurse and scrub table on one side of the patient or the cardiopulmonary bypass machine, associated heater-cooler unit and perfusionist on the other side of the operating table.

When positioned in this way the monitor would be within the sterile field and therefore likely to contaminate the field. Therefore some means of sterile barrier system is required to be positioned between the sterile field and components that are impossible or impractical to sterilise, such as the monitor and its attached cables. This is explained in more detail below.

The monitor itself may be 'Plug-and-Play'. This has obvious benefits in respect of ease of use, ease of training and offers independence of the EVH operator from other operating theatre personnel, as there is no need for the operator or other theatre staff to adjust display settings, avoiding time consuming adjustments during use.

The monitor is light in weight and may therefore be easily handled by one person, such as the EVH operator, who is typically wearing gloves. The operator may thus ensure that all manipulations are performed without contaminating the sterile field, such manipulations including but not limited to assembly of the monitor mount apparatus and deploying the monitor drape over the monitor.

The support arm may comprise a tubular sleeve.

The friction assembly may comprise:
a friction clamp stem adapted to frictionally engage with the distal end of the support arm,
a radial arm fixed to the friction clamp stem and extending radially from the longitudinal axis of the support arm,
a mounting bar fixed to the radial arm and extending spaced from and parallel to the longitudinal axis of the support arm, and
a friction clamp mechanism adapted to frictionally engage the monitor mounting bracket with the mounting bar.

Preferably the friction clamp stem is a quill stem.

The quill stem may comprise a tubular sleeve portion extending from the transverse bar, adapted to fit slidingly within the support arm and having a wedge bearing surface at its end distal from the transverse bar.

The quill stem further may comprise a threaded wedge nut adapted to fit slidingly within the support arm and having a wedge bearing surface adapted to engage the wedge bearing surface of the tubular sleeve portion.

Preferably the wedge bearing surfaces subtend an angle of less than 45° with the longitudinal axis of the support arm, more preferably less than 35°. This has the advantage that there is a relatively large axial movement of the threaded wedge nut against the tubular sleeve portion, and a relatively smaller radial movement.

The quill stem may further comprise a threaded screw member having a hand-engageable knob portion at a first end and a screw thread at a second end engageable in the threaded wedge nut. Preferably the screw thread is a shallow screw thread. Because of the relatively small subtended angle of the wedge surfaces with the longitudinal axis, and the resulting mechanical advantage, a small rotating force on the threaded screw member results in a relatively large clamping force on the inside of the support arm from the quill stem.

As the knob portion is turned further, the clamping force on the inside of the support arm from the quill stem increases. The friction clamp force of the friction clamp stem is thus adjustable by hand rotation of the knob portion by an operator. If the friction clamp force is too high, it can be readily reduced by unscrewing the knob portion.

The friction clamp stem and its components are preferably made of a material which remains dimensionally stable under repeated sterilisation cycles, for example polyoxymethylene (POM) or polyether ether ketone (PEEK).

The monitor mounting bracket typically comprises two bearing apertures through which the mounting bar extends. The bearing apertures allow sliding and rotating of the mounting bar therethrough.

The friction clamp mechanism may comprise:
a clamp screw having a thread engaged in a threaded aperture of the mounting bracket, and
a friction block mounted resiliently to the mounting bracket and engageable with a distal end of the clamp screw,
wherein the friction block is adapted to frictionally engage the mounting bar between the two bearing apertures.

The friction block may comprise a flange extending from a wall of the mounting bracket. It may be a cantilever, with one end fixed to the body of the mounting bracket. It may have a bearing surface adapted to frictionally engage the mounting bar. The bearing surface may be in conformity with the outer surface of the mounting bar.

The clamp screw preferably has a hand-engageable head portion opposite the distal end.

The friction clamp force of the friction clamp mechanism may be adjustable by hand rotation of the head portion by an operator. As a user rotates the head portion clockwise, the clamp screw is driven further towards the friction block, pushing the friction block towards the mounting bar to increase the friction between the friction block and the mounting bar.

The mounting bar preferably extends along the tilt axis.

The friction block may be adapted to permit axial rotation of the mounting bar relative to the friction block under hand operation of a user while the friction block frictionally engages the mounting bar, thereby allowing tilting of the monitor mounting bracket about the tilt axis.

Typically the friction clamp mechanism is adapted to permit said axial rotation under an operating force of less than 20 N applied by the hand of a user, more preferably less than 15 N, most preferably less than 10 N. Typically the force required to move the display monitor is not more than the force which can be applied by one or more fingers of one hand of a user. Typically the friction assembly means that the monitor comes to rest as soon as the force is reduced to beneath a threshold figure, which may be less than 5 N, or of the order of 1 N or 2 N. This means that the display monitor will not move when subject to a slight accidental contact force but is easily moved by an intentional movement applied by the fingers of one hand.

The friction block may be adapted to permit axial movement of the mounting bar relative to the friction block under hand operation of a user while the friction block frictionally engages the mounting bar, thereby allowing translational movement of the monitor mounting bracket along the tilt axis.

The friction clamp stem may be adapted to permit rotation of the radial arm and mounting bar about the longitudinal axis of the support arm under hand operation of a user while the friction clamp stem frictionally engages with the distal end of the support arm, thereby allowing vertical movement of the monitor mounting bracket relative to the support arm.

According to a **second aspect** of the present invention there is provided an operating table arrangement for use in an operating theatre, the arrangement comprising:
an operating table, and
an apparatus according to the first aspect of the present invention,

wherein the mount is attached to the operating table,
wherein the display monitor is positioned in a location suitable for viewing by an endoscopic vein harvesting (EVH) operator using an endoscope, and
wherein the display monitor is adapted to display an image from the endoscope.

Preferably the display monitor has a visible display area and is covered by a sterile monitor drape.

The sterile monitor drape may comprise a tubular plastic sheath of flexible plastic sheet material having an open end and a closed end,
wherein the closed end fits securely over the display monitor, and
wherein the plastic sheath has an aperture which is substantially aligned in size and position with the visible display area, the aperture being closed with a sheet of optically transparent material of stiffness greater than that of the flexible plastic sheet to form an optically transparent window.

The optically transparent window may have a peelable protective membrane thereon to protect the window prior to use of the display monitor.

The sterile monitor drape may include an aperture through which a portion of the friction assembly connecting the monitor mounting bracket to the support arm may be inserted to engage with the monitor mounting bracket when the monitor mounting bracket is secured to the display monitor. The portion of the friction assembly which may be inserted may be the mounting bar of the friction assembly.

In this way the monitor may be non-sterile and protected by the drape, while the friction assembly may be sterile such that it does not need to be protected by the drape.

According to a **third aspect** of the present invention there is provided a method of displaying an image from an endoscope, comprising:
providing an apparatus according to the first aspect, wherein the mount is attached to an operating table,
providing an endoscope,
frictionally clamping the support arm to the mount using the clamp assembly, such that the support arm extending along its longitudinal axis in a horizontal plane parallel to a plane of the operating table, and the display monitor is in a coarse position adjacent to a working location on or adjacent to the operating table so that an image from the endoscope is viewable on the display monitor by a user at the working location,
applying a force by hand operation to the display monitor to provide fine adjustment of the display monitor position, by one or more of:
   tilting of the monitor mounting bracket about a tilt axis parallel to the longitudinal axis of the support arm,
   translational movement of the monitor mounting bracket along the tilt axis, and
   vertical movement of the monitor mounting bracket relative to the support arm.

The method may include the step of grasping and rotating a rotatable adjusting sleeve, either directly or through a sterile drape, to adjust a friction brake of the clamp assembly. The clamp assembly may be operable to a frictionally clamped state in which the support arm is held in a required position by the clamp assembly when no force is applied by a user to the support arm, monitor mounting bracket connected or display monitor, and in which the support arm can be moved under hand operation of a user when sufficient force is applied by the user to the support arm, monitor mounting bracket connected or display monitor.

The method may include the step of adjusting the friction clamp force of the friction clamp stem by hand rotation of the knob portion of the threaded screw member of the quill stem.

The method may include the step of adjusting the friction clamp force of the friction clamp mechanism by hand rotation of the head portion of the clamp screw of the friction clamp mechanism.

The method may include providing fine tilt adjustment of the display monitor position, by axially rotating the mounting bar relative to the friction block under hand operation of a user while the friction block frictionally engages the mounting bar, thereby allowing tilting of the monitor mounting bracket about the tilt axis.

The method may include providing fine horizontal adjustment of the display monitor position, by axially moving the mounting bar relative to the friction block under hand operation of a user while the friction block frictionally engages the mounting bar, thereby allowing translational movement of the monitor mounting bracket along the tilt axis.

The method may include providing fine vertical adjustment of the display monitor position, by rotating the radial arm and mounting bar about the longitudinal axis of the support arm under hand operation of a user while the friction clamp stem frictionally engages with the distal end of the support arm, thereby allowing vertical movement of the monitor mounting bracket relative to the support arm.

The present disclosure also provides a method of performing endoscopic conduit harvesting from a body part. The method, which is not part of the present invention, comprising:
providing an apparatus according to the first aspect, wherein the mount is attached to an operating table,
frictionally clamping the support arm to the mount using the clamp assembly, such that the support arm extending along its longitudinal axis in a horizontal plane parallel to a plane of the operating table, and the display monitor is in a coarse position adjacent to an incision site in the body part on or adjacent to the operating table,
making an incision at the incision site,
introducing a port into the incision,
introducing an endoscope into the port,
introducing a conduit harvesting tool into the same port or another port,
applying a force by hand operation to the display monitor to provide fine adjustment of the display monitor position, by one or more of:
   tilting of the monitor mounting bracket about a tilt axis parallel to the longitudinal axis of the support arm,
   translational movement of the monitor mounting bracket along the tilt axis, and
   vertical movement of the monitor mounting bracket relative to the support arm.

The method may include the step of effecting conduit harvesting using the conduit harvesting tool.

The method may include the step of grasping and rotating a rotatable adjusting sleeve, either directly or through a sterile drape, to adjust a friction brake of the clamp assembly. The clamp assembly may be operable to a frictionally clamped state in which the support arm is held in a required position by the clamp assembly when no force is applied by a user to the support arm, monitor mounting bracket connected or display monitor, and in which the support arm can be moved under hand operation of a user when sufficient force is applied by the user to the support arm, monitor mounting bracket connected or display monitor.

The method may include the step of adjusting the friction clamp force of the friction clamp stem by hand rotation of the knob portion of the threaded screw member of the quill stem.

The method may include the step of adjusting the friction clamp force of the friction clamp mechanism by hand rotation of the head portion of the clamp screw of the friction clamp mechanism.

The method may include providing fine tilt adjustment of the display monitor position, by axially rotating the mounting bar relative to the friction block under hand operation of a user while the friction block frictionally engages the mounting bar, thereby allowing tilting of the monitor mounting bracket about the tilt axis.

The method may include providing fine horizontal adjustment of the display monitor position, by axially moving the mounting bar relative to the friction block under hand operation of a user while the friction block frictionally engages the mounting bar, thereby allowing translational movement of the monitor mounting bracket along the tilt axis.

The method may include providing fine vertical adjustment of the display monitor position, by rotating the radial arm and mounting bar about the longitudinal axis of the support arm under hand operation of a user while the friction clamp stem frictionally engages with the distal end of the support arm, thereby allowing vertical movement of the monitor mounting bracket relative to the support arm.

### Brief Description of the Drawings

The invention will be described, by way of example only, with reference to the drawings in which:
Fig. 1 shows an EVH operator using a monitor mount apparatus according to an embodiment of the present invention;
Fig. 2 shows the mount, clamp assembly, support arm and mounting bar of the monitor mount apparatus of Fig. 1;
Fig. 3 shows an exploded view of the friction assembly of the monitor mount apparatus of Fig. 1;
Fig. 3A shows the threaded wedge nut of the friction assembly of Fig. 3 in side elevation;
Fig. 4 shows the friction assembly of Fig. 3 prior to insertion in the support arm of the monitor mount apparatus of Fig. 1;
Fig. 5 shows the mount, clamp assembly, support arm, mounting bar and display monitor of the monitor mount apparatus of Fig. 1;
Figs. 6 and 7 show front and rear views of the mounting bracket of the monitor mount apparatus of Fig. 5;
Figs. 8 to 10 are schematic drawings showing the adjustment ranges of the tilt angle, height and horizontal travel respectively of the display monitor and mounting bracket of the monitor mount apparatus of Fig. 5;
Fig. 11 shows an operator inserting the clamp assembly, support arm and mounting bar of the monitor mount apparatus of Fig. 1 into a mount on an operating table;
Fig. 12 shows an operator inserting a display monitor of the monitor mount apparatus of Fig. 1 into a sterile drape;
Fig. 13 shows an operator threading a sterile mounting bar of the monitor mount apparatus of Fig. 1 through a fenestration in the sterile drape of Fig. 12;
Fig. 14 shows an operator removing a protective film from the sterile drape of Fig. 12;
Figs. 15A and 15B show the anti-rotation bush and collet of a collet clamp assembly of a monitor mount apparatus according to an embodiment of the present invention;
Figs. 16A and 16B show a side view and a section respectively of a collet clamp of the collet clamp assembly of Figs. 15A and 15B;
Fig. 17shows an exploded view of the collet clamp assembly of Figs. 15A, 15B, 16A, and 16B;
Figs. 18 and 19 show incision sites used in a method of performing endoscopic conduit harvesting according to the present invention from a leg and an arm respectively;
Fig. 20 shows a port introduced into an incision in a method of performing endoscopic conduit harvesting according to the present invention;
Fig. 21 shows an endoscope for introducing into a port in a method of performing endoscopic conduit harvesting according to the present invention;
Fig. 22 shows a conduit harvesting tool for use in the method of performing endoscopic conduit harvesting according to the present invention;
Fig 23 shows an operating theatre set up for endoscopic harvest of the radial artery from the left arm using a monitor mount apparatus according to an embodiment of the present invention; and
Fig. 24 shows an operating theatre set up for endoscopic harvest of the long saphenous vein from one or both legs using a monitor mount apparatus according to an embodiment of the present invention.

### Description of Specific Embodiments

### Support arm assembly

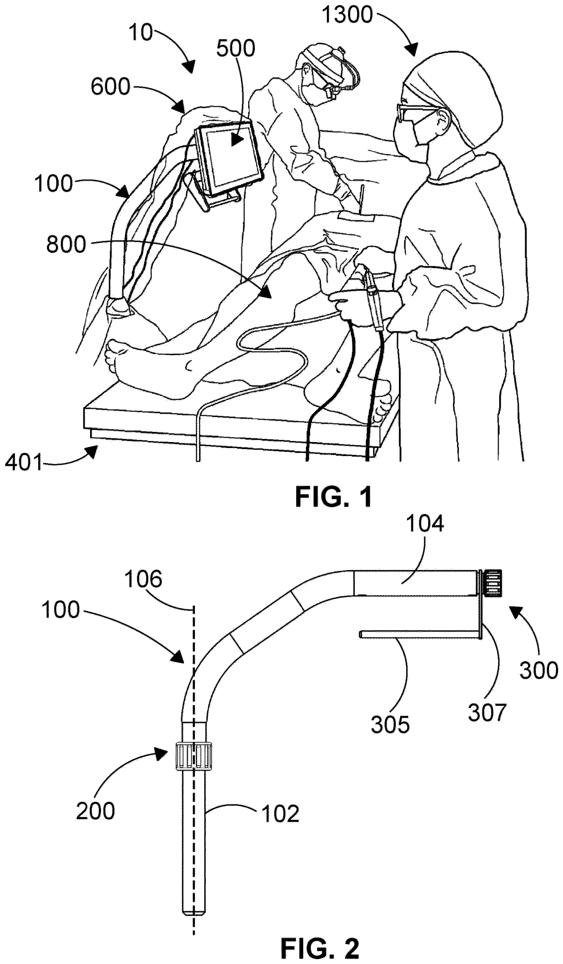

Referring to Figs. 1 to 4, a monitor mount apparatus **10** comprises a display monitor **500** and a support arm assembly **100** provided to support the monitor 500 to provide an on-table monitor for an operating table **401.** The support arm assembly 100 has an upright or vertical portion **102** that may be attached to a reciprocal fitting **402** on an operating table mount 400 and a horizontal support arm **104** that attaches to a display monitor 500.

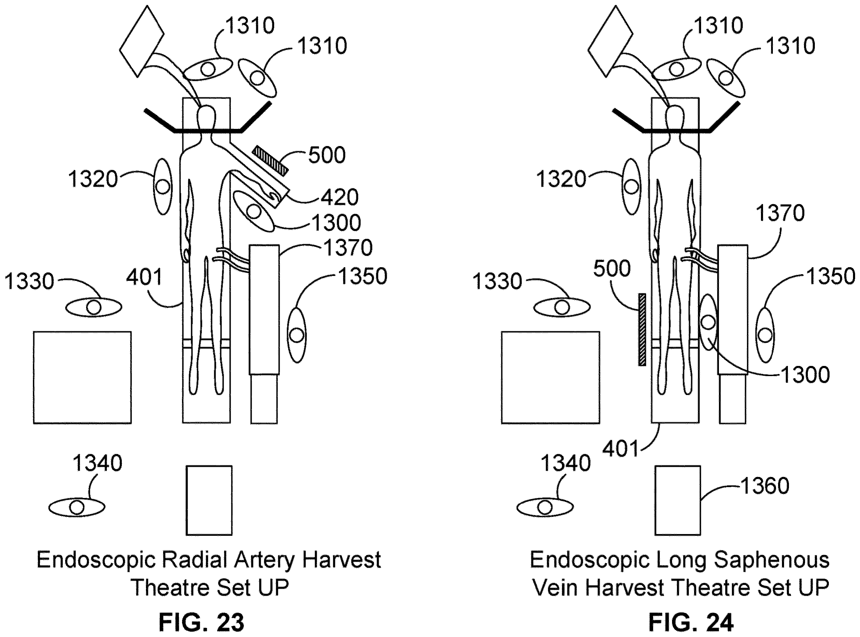

A means of rotating the support arm 104 about the vertical axis **106** of the vertical portion 102 enables the monitor 500 to be positioned perpendicular to the operating table or parallel with the operating table 401, that is along one of the long sides of the table or at some angle in between, according to the choice of the EVH operator **1300.** Two alternative positions can be seen in Figs 23 and 24. These additional angles in between are useful when the EVH operator 1300 is operating at the extremities of the limb, either the foot or the groin. They contribute to ease of use, ease of training and independence of operation by the EVH operator.

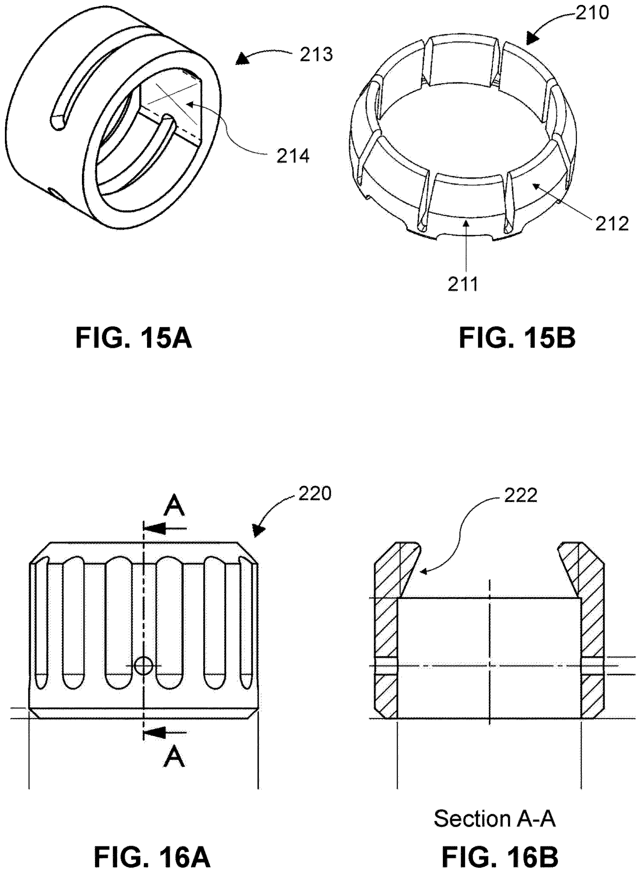

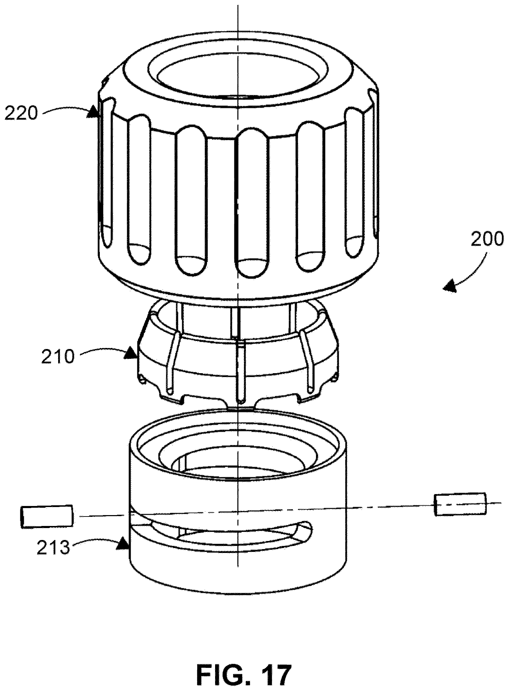

Any suitable means may be used to secure the rotational position of the support arm assembly 100 upon the operating table mount. The illustrated embodiment uses a collet clamp assembly **200.** The collet clamp assembly 200 comprises a collet **210** and collet clamp **220,** illustrated in more detail in Figs. 15 to 17. It is preferably manufactured from materials which can be sterilised between uses, for example by steam sterilisation, so that it may be operated by the EVH operator 1300 within the sterile field. Alternatively it may be of a sufficiently robust design so that it can be positioned on the non-sterile, underside of the sterile drapes, and so that the EVH operator can operate it by grasping it through a standard operating theatre drape, thus maintaining sterility of the EVH operator's gloved hands and ensuring independent operation by the EVH operator without other operating theatre personnel.

The collet clamp assembly 200 functions as a friction brake mechanism. It can be tightened such that the monitor support arm 104 and monitor 500 are held securely in position during use and do not move when fine adjustments are carried out to the tilt angle or position of the monitor 500 or when the monitor 500 or support arm 104 are inadvertently touched or knocked by the scrub nurse, surgeon or other surgical assistant. However the collet clamp is adjustable in tightness, so that the monitor 500 or support arm 104 can moved by the EVH operator 1300 with a firm force of the hand. The use of such a friction brake has the advantage of efficiency and time saving, because there is no need to unlock, reposition and relock to adjust the position of the monitor 500. Prior art locking mechanisms require two hands, one to support the monitor and one to control the locking mechanism. These features therefore contribute to ease of use, ease of training, efficiency and independence from other operating theatre personnel.

In use, the upright or vertical portion 102 of the support arm assembly 100 is inserted through the collet clamp assembly 200 into a reciprocal fitting 402 on the operating table mount 400. The collet clamp assembly 200 may either be above the surgical drape within the sterile field, if it has been sterilised, or below the surgical drape outside the sterile field, if it has not been sterilised. In either circumstance the EVH operator 1300 is able to operate the collet clamp mechanism 200 whilst maintaining the sterility of his or her gloved hands, either directly or through the patient drapes.

The collet clamp assembly 200 incorporates an anti-rotation mechanism so that the collet 210 does not rotate as the collet clamp 220 is screwed onto the collet. This is described with reference to Figs. 15A, 15B, 16A, 16B, and 17. In the illustrated embodiment the anti-rotation mechanism is in the form of a D shaped bush **213** and collet 210. The internal flat surface **214** of the D shaped bush 213 prevents rotation of the collet 210 relative to the operating table mount 400, as the reciprocal fitting 402 to which it is attached has a reciprocal shape.

The wall of the collet 210 is slotted at its inner end to form separate wall sections **211,** each of which ends in a bevelled upper surface or shoulder **212.** The collet clamp 220 is a sleeve which fits outside of the collet 210. The internal surface of the collet clamp 220 has a reciprocal surface **222** which interfaces with the shoulders of the collet. As the collet clamp 220 is screwed onto the collet 210 the shoulders 212 are urged inwards so as to clasp the vertical portion 102 of the support arm assembly 100.

The orientation and arrangement of the collet 210 and collet clamp 220 can be modified or reversed. For example the collet 210 can be rotationally fixed to the support arm assembly 100 instead of the operating table mount 400. The D-shaped anti-rotation bush may be formed as a separate component.

The angle of the bevelled upper surface 212 is sufficiently acute that it self-releases upon unscrewing of the collet clamp 220.

This friction brake mechanism of the collet clamp assembly 200 provides rotational control of the support arm 104 and therefore the monitor about the vertical axis 106 of the vertical portion 102.

The invention is not limited to this particular form of friction brake. A tubular insert portion of the support arm assembly 100 may engage within a tubular sleeve portion of the operating table mount 400, or vice versa. A clamping screw (not illustrated), with a large hand-rotatable screw head, may engage in a threaded radial aperture in the sleeve portion and engage frictionally with the insert portion, to act as a friction brake against relative rotation of the support arm assembly 100 and the operating table mount 400.

The support arm assembly 100 may have additional loops or bars (not shown) for attachment of sutures, clip applicators and other ancillaries used in the course of endoscopic conduit harvesting, to keep these items and tools in close proximity to the operator for ease of use.

The horizontal support arm 104 of the support arm assembly 100 comprises a hollow tube at one end for interface with a friction assembly **300** which supports a monitor mounting bracket **700,** both of which are described below.

### Operating table mount

The operating table mount 400 may be attached to one or both sides of the operating table 401.

The means of attachment to the operating table 401 is preferably by way of an under table frame (not shown) that attaches to both sides of the table for optimal stability during surgery. This is useful in ensuring that the support arm assembly 100 and monitor 500 do not move during surgery.

Clamps (not shown) may attach to side rails commonly found on operating tables, or may grasp the table top directly. The latter mechanism is preferable for attachment to the carbon fibre table tops commonly found on operating tables used in cardiac catherization laboratories and hybrid operating theatres. Fixed side rails are common on the operating tables found in general cardiac operating theatres.

Cross members of the under table frame are preferably made from radiolucent material such as carbon fibre, so that the invention may be used with fluoroscopy in for example a hybrid operating room.

Optionally the support arm assembly 100 may be attached to a linear actuator (not shown). The purpose of the linear actuator is to elevate or lower the height of the monitor 500, thereby adding further coarse control over the position of the monitor.

### Monitor mounting friction assembly

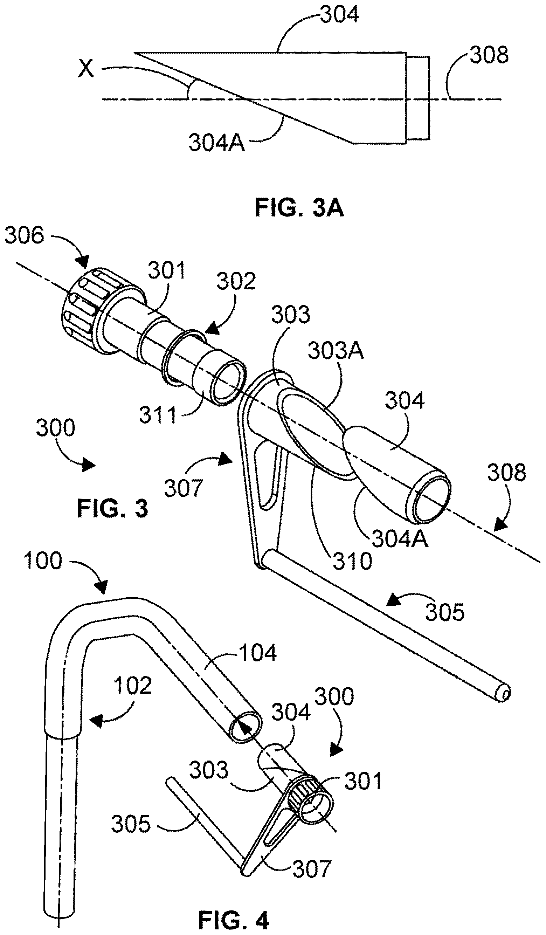

With reference to Figs. 3 to 7, the friction assembly or monitor mounting assembly 300 forms an interface between the support arm 104 and the monitor mounting bracket 700 fixed to the on-table display monitor 500. In its connection with the support arm 104, the friction assembly or monitor mounting assembly 300 incorporates a friction brake mechanism that allows the EVH operator 1300 to adjust the position of the monitor 500. This effectively provides a fine adjustment of height, horizontal position parallel to the support arm 104 and tilt about a horizontal axis of the monitor 500, as explained below.

The friction assembly 300 comprises a friction clamp stem **310** adapted to frictionally engage with the distal end of the support arm 104, a radial arm **307** fixed to the friction clamp stem 310 and extending radially from the longitudinal axis **308** of the support arm 104, a mounting bar **305** fixed to the radial arm 307 and extending spaced from and parallel to the longitudinal axis 308 of the support arm 104, and a friction clamp mechanism **701, 703** adapted to frictionally engage the monitor mounting bracket 700 with the mounting bar 305.

In the illustrated embodiment the friction clamp stem 310 is a quill stem, comprising a tubular sleeve portion **303** extending from the radial arm 307, adapted to fit slidingly within the support arm 104 and having a wedge bearing surface **303A** at its end distal from the radial arm 307. The quill stem also comprises a threaded wedge nut **304** adapted to fit slidingly within the support arm 104 and having a wedge bearing surface **304A** adapted to engage the wedge bearing surface 303A of the tubular sleeve portion 303. The quill stem also comprises a threaded screw member **301** having a hand-engageable knob portion **306** at a first end and a screw thread **311** at a second end, which engages with a corresponding internal thread of the threaded wedge nut 304. A low friction washer **302** is provided on the screw member 301, to allow the screw member 301 to rotate easily when the knob portion 306 bears against the tubular sleeve portion 303.

In operation the threaded wedge nut 304 is drawn towards the wedge bearing surface 303A of the tubular sleeve portion 303 by rotation of the threaded screw member 301.

The wedge bearing surfaces 303A, 304A subtend an angle X of less than 45° with the longitudinal axis 308 of the support arm, as shown in Fig. 3A. In practice it has been found that an angle X of less than 35° is effective. This has the advantage that there is a relatively large axial movement of the threaded wedge nut 304 against the tubular sleeve portion 303, and a relatively smaller radial movement, so there is a mechanical advantage, allowing the knob portion 306 to be turned by hand, and yet achieving a relatively high clamping force between the quill stem and the support arm 104. A further mechanical advantage is achieved by making the screw thread 311 a shallow screw thread.

As the knob portion 306 is turned further, the clamping force on the inside of the support arm 104 from the quill stem increases. The friction clamp force of the friction clamp stem is thus adjustable by hand rotation of the knob portion 306 by an operator 1300. If the friction clamp force is too high, it can be readily reduced by unscrewing the knob portion 306.

The friction clamp stem 310 and its components are preferably made of a material which can be compressed against a metal surface so as to create a frictional contact between the two surfaces, and which remains dimensionally stable under repeated sterilisation cycles, for example polyoxymethylene (POM) or polyether ether ketone (PEEK). If the components are sterilised, the friction clamp stem can be operated within the sterile field and therefore by the EVH operator 1300.

PEEK is preferred as the material for the wedge nut as it exhibits greater dimensional stability than POM during the process of steam sterilisation.

The knob portion 306 can be tightened sufficiently so that the friction clamp stem 310 holds the monitor 500 securely in position, but not so tight that the monitor 500 cannot be moved by intentional finger force of the EVH operator 1300. In this way, the height of the monitor 500 can be adjusted without having to go through the operation of loosening the screw thread with a tool whilst supporting the monitor and then adjusting position of the monitor and then tightening the screw thread whilst still supporting the monitor, all of which take two hands and possibly even a second person. Thus the operation is entirely within the capability of the EVH operator 1300 alone, and can be done with one hand only. The apparatus of the present invention can be set to counter-balance the weight of the monitor 500 and resist movement of the monitor 500 under gravitational forces but allows the EVH operator 1300 to adjust the position of the monitor 500 with one hand, without having to loosen and tighten the wedge nut repeatedly.

The radial arm 307 is rigidly secured to the tubular sleeve portion 303 and extends perpendicular to the axis 308 of the quill stem 310. In this way the quill stem 310 provides angular adjustment about the axis of the quill stem 310 which, depending upon the orientation of the quill stem 310, can be used to provide a fine height adjustment of the monitor 500.

The monitor mounting friction assembly 300 thus provides a means of support for the monitor 500 and provides a fine height adjustment of the monitor 500. Height adjustment of the monitor 500 is desirable in order to accommodate EVH operators of different stature and also because the operating table may be raised or lowered multiple times during the operative procedure or tipped to the right or left, especially for example during harvest of the internal thoracic arteries by another surgeon working simultaneously.

### Monitor mounting bracket

A monitor mounting bracket **700** is attached to the rear surface **502** of the on-table monitor 500. The monitor mounting bracket 700 may be either sterile or non-sterile. The monitor mounting bracket 700 acts as an interface between the horizontal mounting bar 305 of the sterile monitor mounting friction assembly 300 and the non-sterile monitor 500.

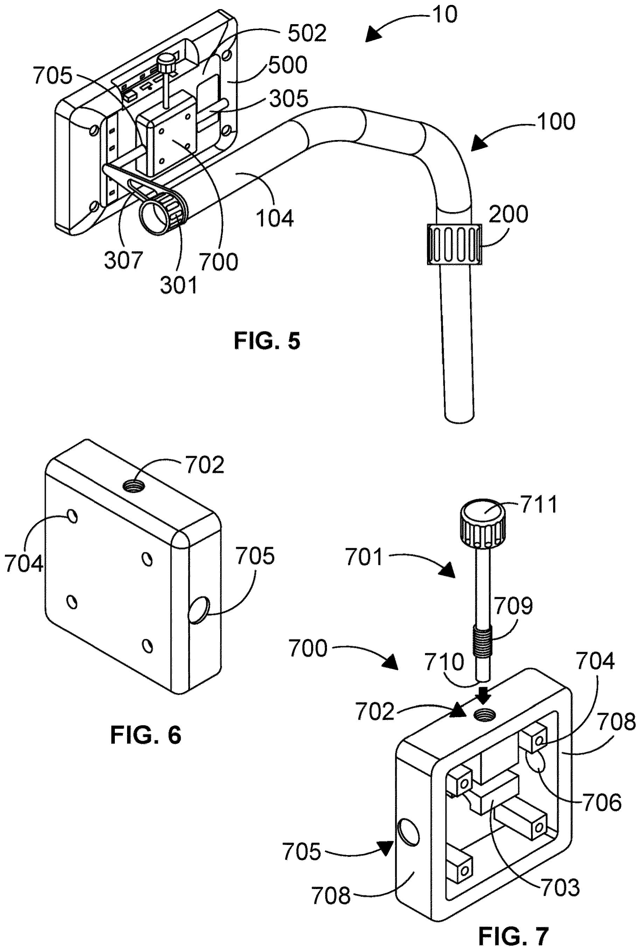

The monitor mounting bracket 700 enables fine tilt adjustment of the monitor position about a horizontal axis for an optimal viewing angle of the screen of the monitor 500 by the EVH operator 1300. The monitor mounting bracket 700 includes a friction brake, which in the illustrated embodiment of Figs. 5 to 7 is a friction clamp mechanism 701, 703 adapted to frictionally engage the monitor mounting bracket 700 with the mounting bar 305.

The monitor mounting bracket 700 is typically a moulded plastic box which can be affixed to the rear of the monitor 500. The bracket 700 has two opposed side walls **708.** Each side wall 708 includes a bearing aperture **705,** 706 through which the mounting bar 305 extends, as shown in Fig. 7. The bearing apertures 705, 706 allow sliding and rotating of the mounting bar 305 therethrough.

The friction clamp mechanism 701, 703 comprises a clamp screw 701 having a thread **709** engaged in a threaded aperture **702** of the mounting bracket 700, and a friction block 703 mounted resiliently to the mounting bracket 700 and engageable with a distal end **710** of the clamp screw 701. The friction block 703 is arranged between the two bearing apertures 705. In the illustrated embodiment of Fig. 7 the friction block 703 is formed as a moulded cantilever extending from the internal surface of mounting bracket 700, although it is to be understood that any appropriate structure, shape and material may be selected for the friction block 703.

The friction block 703 may have a bearing surface adapted to frictionally engage the mounting bar 305. The bearing surface may have a suitable friction coating.

The clamp screw 701 has a hand-engageable head portion **711** opposite the distal end 710. The friction clamp force of the friction clamp mechanism 701, 703 may be adjustable by hand rotation of the head portion 711 by an operator 1300. As a user rotates the head portion 711 clockwise, the clamp screw 701 is driven further towards the friction block 703, pushing the friction block 703 towards the mounting bar 305 to increase the frictional force between the friction block 703 and the mounting bar 305.

The friction clamp mechanism 701, 703 serves as a friction brake, such that when the friction brake is applied there is sufficient frictional force to prevent the monitor 500 from moving under the weight of gravity alone, yet not so much that it cannot be moved purposefully with one hand by the EVH operator 1300 from one position to another, where it continues to be held with sufficient frictional force that it does not move under its own weight. This important feature means that the operator does not need to unlock and then relock with each adjustment of monitor position during the EVH procedure, which saves time. Adjustment can be performed with one hand, which adds further efficiency to the EVH procedure.

The monitor mounting bracket comprises four threaded holes **704** by which screws (not shown) are used to attach the monitor mounting bracket 700 to the monitor 500. The threaded holes may be provided in a VESA variety of configurations for attachment to a range of different types of monitors. The distal end 710 of the clamp screw 701 rests on the friction block 703 located on an internal, unseen surface of the monitor mounting bracket 700. The lower surface of the friction block 703 is optionally curved in profile, the concavity matching a reciprocal convexity of the mounting bar 305.

One or both of the bearing apertures 705, 706 may have a concave 'lead in' for the mounting bar 305, which may also serve to prevent any patch material surrounding a fenestration in a monitor drape used to cover the monitor 500 from becoming drawn into the mounting bracket 700 during passage of the mounting bar 305 through the bearing aperture 705, 706 and into the mounting bracket 700.

The mounting bracket 700 is made from a suitable resilient material such as polyoxymethylene (POM) or PEEK. The friction block 703 may be formed of the same resilient material, so that it may be compressed against the mounting bar 305 to hold the monitor mounting bracket 700 and monitor 500 in position. The EVH operator 1300 may tighten the clamp screw 701 at the start of the procedure during set up, and then should not need to adjust the mechanism again. During the procedure he or she can adjust the monitor position with one hand by an intentional finger force sufficient to overcome the frictional forces between the mounting bar 305, the friction block 703 and the bearing apertures 705, 706.

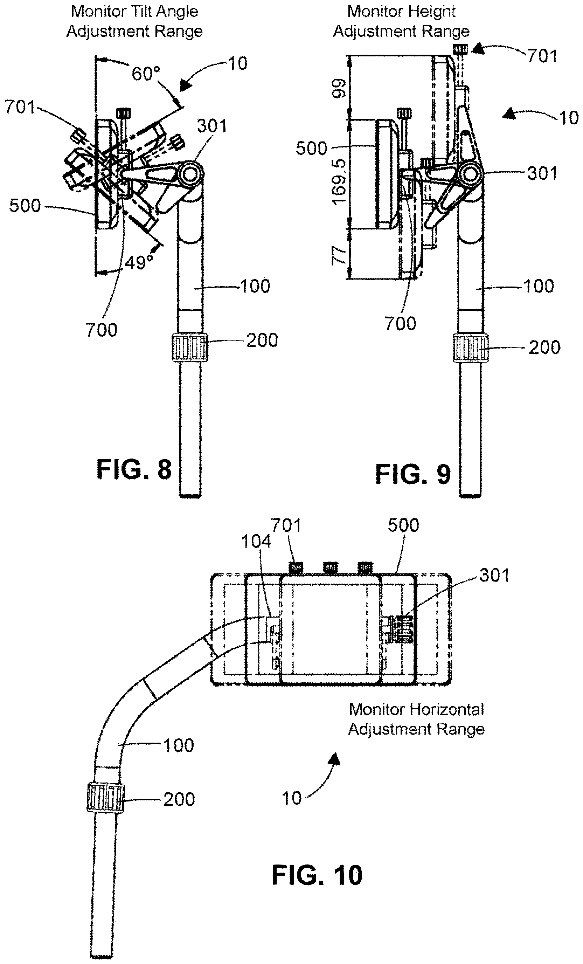

By pushing the top of the monitor 500 forwards or backwards, the mounting bar 305 rotates past the friction block 703, allowing fine adjustment of the horizontal tilt position of the monitor 500, as illustrated in Fig. 8, which shows the two end tilt positions and an intermediate tilt position superimposed.

By pushing the monitor 500 upwards or downwards, the radial arm 307 rotates about a horizontal axis relative to the support arm 104, allowing fine adjustment of the vertical position of the monitor 500, optionally with simultaneous tilt adjustment, as illustrated in Fig. 9, which shows the two end vertical positions and an intermediate vertical position superimposed.

By pushing the monitor 500 sideways, the mounting bar 305 slides past the friction block 703, allowing fine adjustment of the horizontal position of the monitor 500, as illustrated in Fig. 10, which shows the two end horizontal positions and an intermediate horizontal position superimposed.

### Drape

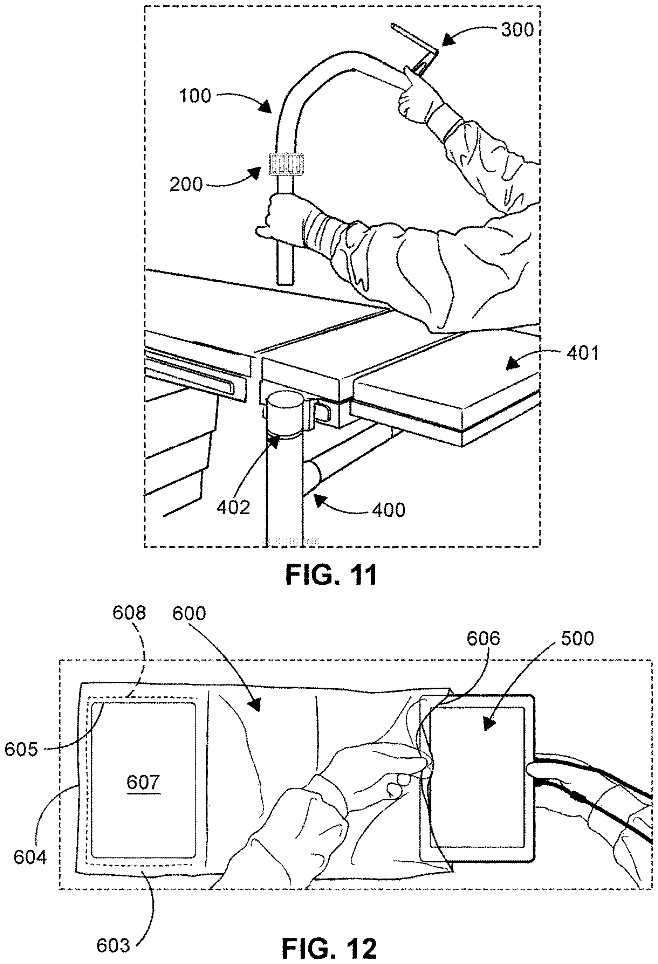

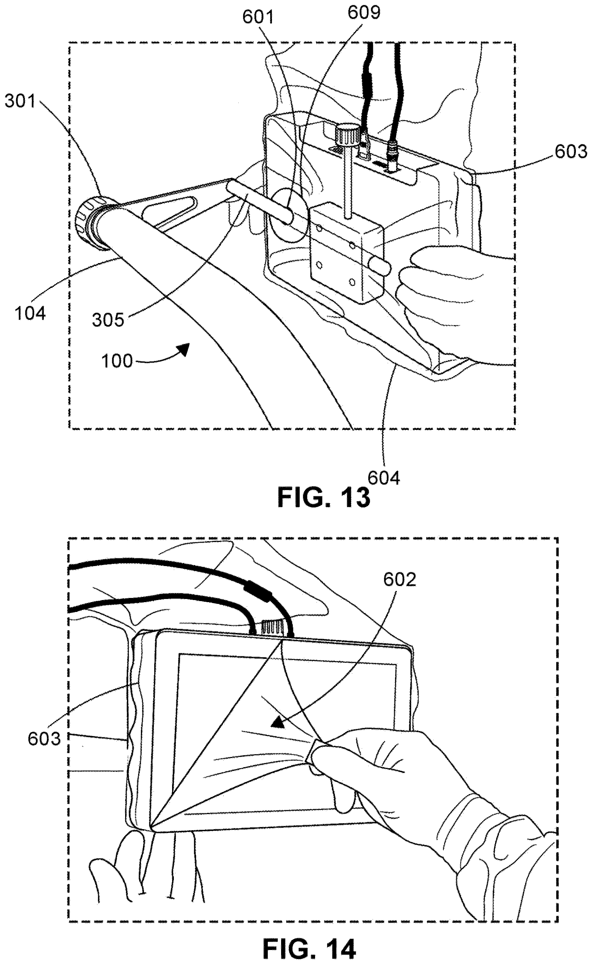

The monitor 500 of this disclosure may be provided with a sterile disposable monitor drape to maintain sterile barrier between the monitor and the surgical field and allows the EVH operator to adjust the position of the monitor himself or herself quickly and easily without having to ask for the assistance of circulating operating theatre personnel. The drape is described with reference to Figs. 11 to 14

Before the monitor 500 is fitted to the support arm assembly 100, the support arm assembly 100 is connected by the clamp assembly 200 to the mount 400 attached to the operating table 401, as shown in Fig. 11.

The monitor drape **600** can be seen in Figs. 12 to 14 and comprises a sheath made from a suitable material like polyethylene sheeting that may be formed as lay-flat tubing. The monitor drape 600 has a closed end **604** and an open end **606.**

The monitor drape 600 has a pocket **603** at the end of drape configured so that it stretches over the monitor 500, sufficiently tight that it holds the monitor drape securely in position during the procedure, specifically so that it does not slide off the monitor under its own weight but not so tight that it tears when the monitor is pushed into the pocket 603.

The monitor drape 600 is manufactured from a suitable material such as polyethylene which is easily obtained in convenient sizes and easily sterilised by ethylene oxide or gamma irradiation so that the monitor drape is sterile at point of use

The monitor drape 600 incorporates has a rectangular aperture or fenestration **605** at one end to which is secured an optically transparent window **607** for clear viewing of the screen of the monitor 500 by the EVH operator.

The optically transparent window 607 may be secured to the lay-flat tubing by any suitable means, such as adhesive or strips of double sided tape **608** or a gasket cut to the shape of the margins of the fenestration in the lay flat tubing.

The monitor drape may incorporate an opaque screen border (not shown) to shield from view the means of joining 608 the optically transparent window 607 to the lay-flat tubing or main body of the drape 600.

The optically transparent window 607 is provided with a peel away protective membrane **602** that is removed by the EVH operator at the start of the procedure and protects the window during, manufacture, storage and setup.

The monitor drape 600 is sterilised with a suitable method of sterilisation such as exposure to ethylene oxide or gamma irradiation.

The monitor drape 600 may be folded in such a way that can be packaged conveniently for sterilisation and for ease of transfer into the operative field upon opening the packaging in such a manner as to preserve the sterility of the drape inside the pack

The monitor drape 600 may, furthermore, be folded in such a way that it may be deployed over the monitor and over the power and signal cables attached to the monitor in a sterile manner by two theatre operatives, a sterile operative that holds the monitor drape open and a non-sterile operative that passes the monitor, power and signal cables into the drape. In this way a sterile barrier is maintained between the monitor, signal and power cables and the operative field.

The sterile monitor drape 600 includes an aperture or fenestration **601** on the opposite side of the pocket 603 to the window 607, through the mounting bar 305, which connects the monitor mounting bracket 700 to the support arm 104, which may be inserted to engage with the monitor mounting bracket 700 when the monitor mounting bracket 700 is secured to the display monitor 500. In this way the monitor 500 may be non-sterile and protected by the drape 600, while the friction assembly 300 may be sterile such that it does not need to be protected by the drape.

The monitor drape 600 may include an aperture or fenestration 601 on the left or right or both sides through which a sterile mounting bar 305 or other means of attachment may be inserted.

Optionally, a patch **609** may be provided around the aperture or fenestration 601. The patch 609 may be made form a reinforcing material, i.e. material that is stronger in tensile strength and tear resistance than the polythene sheeting, so that the fenestration does not tear, and so that it holds its shape around the mounting bar 305 as it is passed through. In this way a sterile barrier is maintained between the two distinct zones of sterile field and non-sterile field on either side of the aperture 601.

The monitor drape 600 may incorporate a 3D filter (not shown) located in front of the surgical display monitor 500. The 3D filter may comprise a thin substrate, such as glass, upon which one or more optically active layers are applied. The filter is thus mounted in front of the surgical display to act as a beam splitter for light exiting the panel of the surgical monitor 500. The filter ensures that the image content displayed through the monitor drape is spatially perceived by the observer.

### Endoscopic tools

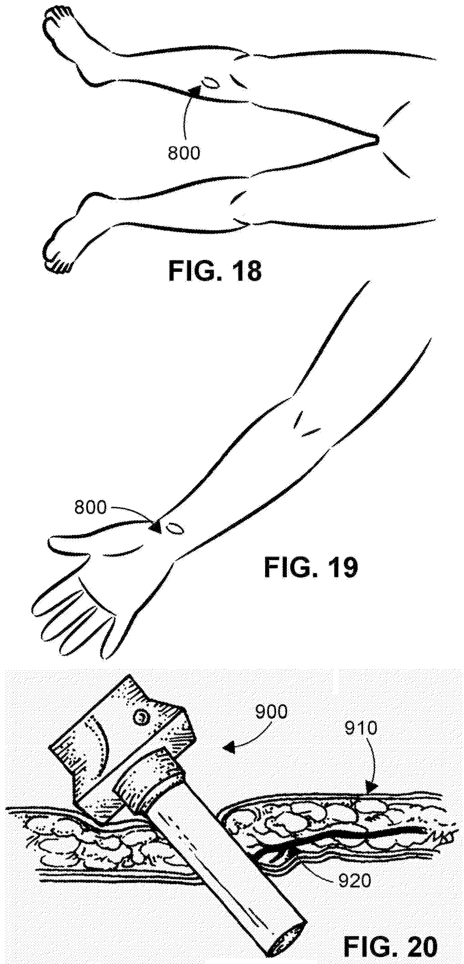

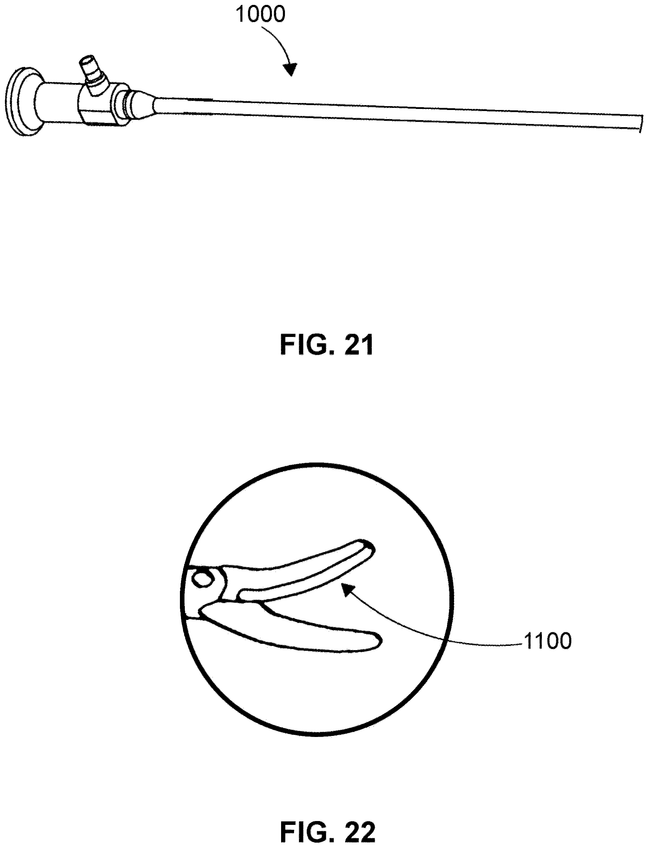

Referring to Figs. 18 to 22, the set of kit that enables conduits to be harvested endoscopically includes a set of endoscopic tools. The endoscopic tools comprise an endoscope **1000,** an effector tool or tools **1100** for dissecting the conduit from surrounding anatomical structures and /or for dividing and securing haemostasis of side branches either by cautery, clips or in some other way; and a port **900** for providing ease of passage of endoscope and/or effector tools through the skin **910** and subcutaneous tissues **920.**

The endoscope 1000 is of suitable length and design that the conduit to be harvested may be imaged along its entire length. The effector tool or tools may include graspers, cutters, clippers and cauterising elements 1100.

The port 900 may be simple open hollow tube with a removable obturator for its insertion through the body surface or otherwise may be equipped with one or more seals so that the body cavity or tissue space can be distended with gas or fluid. The port may incorporate multiple channels for both endoscope and one or more effector tools and/or have other unique features for the efficient docking and orientation of endoscope and/or effector tools or tools.

### Carrying out the invention

The invention and method of use brings the screen of a monitor 500 into the operative field for ease of use by the operator 1300 during endoscopic conduit harvesting, creating in this way an 'on table monitor'. Such a monitor has not hitherto been available in the field of endoscopic conduit harvest.

Referring to Figs. 23 to 24, Fig. 23 shows an operating theatre set up for endoscopic harvest of the radial artery from the left arm using the monitor mount apparatus 10. The left arm is supported on an arm board **420** and display monitor 500 disposed opposite the EVH operator 1300. The monitor 500 may be positioned above the arm, with the support arm 104 (not shown in Fig. 23) extending along its longitudinal axis in a horizontal plane parallel to the plane of the arm board 420, which may itself be affixed perpendicular to the main operating table 401 or more preferably at an acute angle to the operating table 401, so as to reduce stress on the shoulder during surgery.

Fig 24 shows an operating theatre set up for endoscopic harvest of long saphenous vein from one or both legs using the monitor mount apparatus 10. The display monitor 500 is disposed opposite the EVH operator 1300, with the support arm 104 (not shown in Fig. 23) extending along its longitudinal axis in a horizontal plane parallel to the plane of the operating table 401.

In the operating theatre set up of both Fig 23 and Fig. 24 the EVH operator 1300 may adjust the position of the monitor using the monitor mount apparatus 10 described above, without interference with or by the other personnel in the operating theatre, who may include anaesthetists **1310,** surgeon **1320,** scrub nurse **1330,** circulating nurse **1340** and perfusionist **1350,** and without interference with or by other apparatus in the operating theatre, such as an imaging stack and CO2 insufflator **1360** and CPB machine and heater/cooler **1370.**

The apparatus of the present disclosure includes a number of friction brake mechanisms for optimal adjustment of the position of the on-table monitor, preferably three separate, but cooperable brake mechanisms. Common features of these three separate friction brake mechanisms include a deformable material compressible against a solid material, a screw thread to set and control the frictional contact between the deformable material and solid material the effects of which are to allow operation by one hand by the EVH operator independent of other personnel and by one hand only. Materials have been selected so that the devices are capable of being sterilised. Finally operation of each is intuitive by nature of consistency of operation and grooved outer surface of the knobs sitting atop each screw head mechanism.

In each case the operator is able to adjust the screw mechanism in such a manner as to secure the position of the monitor during use but not so tightly that it that cannot be overcome by intentional movement by the operator with one hand. In this way the precise position of the monitor may be adjusted quickly and easily by one hand without having to go through the process of unlocking, adjusting and relocking every time.

The various friction brake mechanisms cooperate with each other to provide means of very fine and very coarse control of position with simple intuitive operation to provide the perfect position for the individual operator at all times during the operation.

A first friction brake allows the screen to be rotated slightly about this axis for optimal viewing especially at the extremities of the graft dissection where the surgeon may choose to change his position slightly in such a way that he is facing more towards the head or more towards the feet.

A second and third friction brake allows the surgeon to rotate the screen for his seated or standing height and, if the table top has been rotated, about this axis for better exposure of the harvest site. As with radial artery harvest, during the procedure the position of the table may change due to other surgeons harvesting grafts simultaneously. In a typical example the LITA may be harvested by another surgeon who may choose to rotate the table top away from him and elevate the table top so as to provide optimal view for LITA harvest through the open sternotomy incision. The operator may then adjust the position of the monitor by hand movement without having to unlock and relock the brake.

A fenestration and method of use of said fenestration is provided as a means of separating sterile components of the invention from non-sterile components of the invention without compromising the sterile operative field and whilst allowing the operator to have control of all of these various components of the invention without having to resort to assistance from other operating theatre personnel. Fenestration is provided in the monitor drape. Use may be made of fenestration made in the patient drapes for insertion of the support arm and, optionally, the collet clamp assembly.

The disclosure includes a method of using a fenestration in the sterile barrier system or medical drape as a means of maintaining two distinct fields or zones a sterile field or zone and a non-sterile field or zone. A portion of the sterile device is passed through the drape to the non-sterile side where it interfaces with a reciprocal fitting on the other side of the non-sterile portion of the device.

This is useful because it is inefficient to sterilise all parts of the system. For example, the operating table mount does not need to be sterile to serve its function, if protected from the sterile zone by a physical barrier or drape. It may be impractical to sterilise parts of the apparatus of the present invention between cases, such as the on table monitor.

Training in endoscopic conduit harvesting is difficult and there is a significant learning curve required to master the technique. A dearth of fully trained and competent operators is a major factor hampering widespread adoption of endoscopic conduit harvesting, despite its obvious advantages. Furthermore, conduit harvesting is often not performed by doctors but actually by surgical care practitioners, who, unlike surgeons, may not have trained in the various disciplines of surgery, or who have flexibility and experience from other fields of surgery to bring to bear on the procedure. In any event training of surgical care practitioners is considered to take longer than training of surgeons, and the training of surgeons already carries a significant learning curve associated with endoscopic conduit harvesting.

Sickness absence is common amongst surgical care practitioners performing conduit harvesting on a frequent, often twice daily, basis. Repetitive strain injuries are thought to be the main cause of sickness absence in this professional group. It is considered that endoscopic conduit harvesting using this new improved technique will reduce repetitive strain injury because the operator is able to work more quickly and more comfortably on account of the extensive control over monitor positioning provided by the components of this kit and the various mechanisms for adjustment described in this invention and the independence of the operator from other theatre personnel in making these coarse and fine adjustments.

### A method of use

The present disclosure includes a method having one or more of the following steps, in any combination. In particular any one or more of the following method steps may be combined with the steps of a method according to the third or fourth aspects of the present invention, or with a method of using the apparatus according to the first aspect of the present invention, or with a method of using the operating table arrangement according to the second aspect of the present invention.

In this technique, as applied to the endoscopic harvest of long saphenous vein for coronary artery bypass grafting, the operator preferably stands or sits on one side of the table, most often to the patient's left side. The support arm and ultimately the on table monitor is located along the opposite side of the table and oriented towards the operator for optimal viewing.
- A patient is positioned on an operating table and prepared for a coronary artery bypass grafting (CABG) operation.
- An operating table mount is attached to the operating table.
- The operating table mount is optionally attached to both sides of the operating table for additional stability.
- A linear actuator is optionally attached to the operating table mount and interposed between the support arm and the operating table mount for additional, very substantial height adjustment.
- The patient's skin is prepared with antiseptic solution and covered in sterile patient drapes in such a manner as to expose a body part for surgery.
- The body part includes at least one of a chest, abdomen, leg and arm for surgery.
- A fenestration is optionally made in said sterile patient drapes to pass a support arm.
- Optionally a collet clamp assembly is attached to the operating table mount.
- The support arm is passed through said fenestration in sterile drapes in a sterile manner to connect with the operating table mount and collet clamp assembly.
- The support arm is positioned over said body part from which conduit will be harvested and monitor and monitor drape assembly adjusted in such a manner that the on table monitor is positioned in the line of sight of the operator, this being the chosen position.
- The collet clamp of the collet clamp assembly is tightened to maintain the position of the support arm in the chosen position, but not so tight that it cannot be moved with intentional forceful movement by one hand.
- A friction assembly is attached to the support arm.
- The friction assembly is tightened to maintain the position of the monitor in the line of sight of operator, but not so tight that it cannot be moved with intentional forceful movement by one hand.
- An on-table monitor is connected with a power source and a signal source.
- A sterile monitor drape is placed over the on table monitor in such a sterile manner as to maintain sterility of the external surface of the monitor drape and drawn out by a circulating nurse.
- The on table monitor is connected to a power source and a signal source.
- The monitor drape is held open by a medical practitioner or nurse that is scrubbed.
- The on table monitor is placed inside the monitor drape by a nurse that is not scrubbed (circulating nurse).
- The on table monitor is pushed to the end of a monitor drape.
- The on table monitor is slid into a pocket at the end of the monitor drape that is formed by two restrictions in the lateral dimensions of the monitor drape.
- The monitor drape is drawn over the power and/or signal cables by the circulating nurse so as to create a sterile sheath for the power and/or signal cables and separate them from the sterile field.
- The mounting bar of the friction assembly is passed through a fenestration in the monitor drape.
- The mounting bar of the friction assembly is passed through the monitor mounting bracket that is applied to the rear surface of the on table monitor.
- The monitor mounting bracket is secured with a clamp screw but not so tight that it cannot be moved with intentional forceful movement by one or more fingers of one hand.
- A protective cover is removed from the monitor drape to reveal an optically transparent window through which the monitor may be viewed whilst maintaining a sterile barrier between the monitor and the surgical field.
- The monitor drape incorporates an optically transparent window.
- The optically transparent window of the monitor drape is protected by a peel off protective cover.
- The assembly comprising the on table monitor, the monitor mounting bracket and the monitor drape is attached to the rest of the friction assembly.
- The protective cover overlying the optically transparent window of the monitor drape is peeled off.
- The height of the on table monitor is adjusted for optimal viewing by height adjusting the lifting mechanism
- An incision is made in the body part
- A port is introduced into the incision.
- An endoscope is introduced into the port.
- A conduit harvesting tool is introduced into the same port or another port
- The endoscope is connected to a camera control unit which is connected with the on table monitor.
- The on table monitor is configured so that the image appears automatically when connected with a power source and connected with a signal source without requiring any further image adjustment by the operator i.e. plug and play.
- The port is optionally connected with a source of CO2 gas under pressure
- CO2 is optionally instilled into the tissue space surrounding the conduit via the port.
- Branches of conduit are divided, said division being aided by visualisation of the same on the on table monitor seen through the optically clear window of the monitor drape, all of the above being positioned above the body part for optimal viewing by the operator.
- The position of the on table monitor is adjusted by the operator for optimal viewing one or more times during the procedure without unlocking and unlocking of the one or more of the friction brake mechanisms.
- The conduit is an artery or a vein.
- The proximal extremity of the conduit is visualised on the monitor and optionally divided for use as a free graft or left attached for use as a pedicled graft.
- The distal extremity of the conduit is visualised on the on table monitor and divided.
- The conduit is removed from the body part, branches are examined and branches optionally secured with ligatures or clips.
- All such preparations made as may be required to render said conduit suitable for use as a conduit for CABG operation, including trimming, cutting, tying, applying clips.
- The harvest site is checked for haemostasis and any additional haemostasis secured.
- A drain is optionally placed, its precise position being guided by visualisation on the same on the monitor seen through the optically clear window of the monitor drape, all of the above being positioned above the body part for optimal viewing by the operator.
- The incision in the body part is closed.
- The on table monitor and monitor mounting bracket is disassembled from the remainder of the friction assembly.
- The monitor drape is disassembled from the surgical monitor and monitor mounting bracket and the drape is discarded.
- The friction assembly is released and disassembled from the support arm.
- The collet clamp assembly is released and support arm disassembled from the operating table mount.
- The friction assembly and support arm are reprocessed by cleaning and sterilisation.
- The linear actuator is optionally disassembled from the operating table mount and support arm or left in situ for the next case.
- The operating table mount is optionally disassembled from the operating table or left in situ for the next case.
- The endoscope and camera control unit are optionally configured to produce a 3D signal.
- The on table monitor is optionally configured to display a 3D image signal

### Other uses

The principles of EVH may equally be applied to harvest of arterial conduits as well, such as radial artery from the arm or internal thoracic arteries from left or right sides, especially if done through a limited chest incision and/or thoracoscopically. Potentially, a gastroepiploic artery could also be harvested from the abdomen using this screen although this conduit is much less commonly used in surgery today.

The set of kit enables the radial artery to be harvested endoscopically. Using similar set of tools to endoscopic vein harvest the surgeon may be seated at the end of the arm / hand and facing towards the shoulder. The monitor may be positioned above the elbow, shoulder or upper arm. Position adjusted using the three friction brakes for optimal comfort during viewing.

Alternatively, with the arm supported on an arm board, the monitor may be positioned above the arm, with the support arm extending along its longitudinal axis in a horizontal plane parallel to the plane of the arm board which may itself be affixed perpendicular to the main operating table or more preferably at an acute angle to the operating table so as to reduce stress on the shoulder during surgery, and with the surgeon standing or seated opposite the monitor.

During the procedure the position of the table may change due to other surgeons harvesting grafts simultaneously, In a typical example the LITA harvested by another surgeon who may choose to rotate the table top away from him and elevate the table top so as to provide optimal view for LITA harvest through the open sternotomy incision. The operator may then adjust the position of the monitor by hand movement without having to unlock and relock the brake.

The set of kit enables LITA and RITA to be harvested endoscopically for CABG or other surgical uses. The endoscopic tools comprise and endoscope, a grasper and an effector instrument such as scissors, diathermy or harmonic scalpel. Clip applicator may also be used and interchanged with the effector instrument. In this technique an endoscope is introduced into the left side of the chest in the 5th or 6th intercostal space. A grasper is introduced through a port in the lower chest wall and operated using the left hand and a dissector, either diathermy or harmonic scalpel are introduced through separate ports. The surgeon sits at the side of the table and the on table monitor is positioned in such a way that it is directly opposite the surgeon just above the chest in the line of sight of the operator and in such a way the visual and motor axes are substantially aligned. The pleura is entered and CO₂ optionally instilled into the pleural space. The operating table top may be tipped to the left or right and/or into the Trendelenburg or reverse Trendelenburg positions in order to use gravity to move viscera away from the anterior chest wall where the LIAT and RITA are located. The friction brakes on the kit allow the table top to be moved in this way without monitor arm moving. Slight adjustment after table top manoeuvres is easily done by hand without hang to unlock and relock the brakes. This provides efficiency of movement and ease of operating. The LITA or RITA can be detached and used as free grafts or else left attached proximally for use as pedicled grafts.

Arterial and venous conduits may be useful in other fields of surgery. They may for example be used for bypass of lower limb vessels in peripheral vascular surgery such as femoro-popliteal bypass grafting where reversed long saphenous vein is typically used to construct a bypass between the femoral artery at the groin and the popliteal artery at the knee. This operation is indicated for patients with obstructive or occlusive disease of the superficial femoral artery that connects these two vessels in the normal anatomy. For more distally placed narrowings or occlusions of the lower limb arteries, conduits may be used for so called, femoro-distal anastomoses or popliteo-distal anastomoses if suitable inflow can be established for the popliteal artery

Arterial and venous conduits harvested by the new improved endoscopic harvesting technique may also be used in plastic and reconstructive surgery.

In all of the above surgeries, there are clear advantages to harvesting conduits through much less invasive incisions using the new improved endoscopic techniques described herein.

### Composition of the surgical kit for endoscopic conduit harvesting

The present disclosure includes a surgical kit including one or more of the following items or components, in any combination. In particular any one or more of the following items or components may be combined with the apparatus according to the first aspect of the present invention, or with the operating table arrangement according to the second aspect of the present invention.

A operating table mount that may be attached to an operating table, optionally a linear actuator, a collet clamp assembly, sterile drapes that may be applied to a patient in such a manner as to expose a body part for surgery (the body part includes at least one of a chest, abdomen, leg and arm for surgery), optionally a fenestration is optionally made in said sterile drapes to pass a support arm, an on table monitor that may be positioned within the surgical field so that it is in the line of sight of the operator, this being the chosen position, a monitor mounting bracket that is attached to the lifting arm, the monitor mounting bracket being adjustable to accommodate positioning for line of sight of operator, a monitor mount comprising block with screw holes that may be attached to the rear surface of a monitor (e.g. VESA configuration), a central bore through which the arm from a monitor mounting bracket may be passed, a clamping screw that may be operated through the monitor drape, a thread in the monitor mount that accepts the clamp screw and allows its distal end to sit against one side of a flange, the flange that may be compressed against the arm of the monitor bracket by turning of the clamp screw so as to secure the position of the monitor on the monitor bracket but not so tight that the monitor cannot be moved by hand to a new position without having to loosen and tighten the clamp screw an on table monitor that may be connected with a power source and a signal source, a sterile monitor drape (with all of the features of outlined below) a port, a conduit harvesting tool that may be introduced into the incision, through the port, the on table monitor being configured so that the image appears automatically when connected with a power source and connected with a signal source without requiring any further image adjustment by the operator i.e. plug and play, the port is optionally being connected with a source of CO2 with CO2 is optionally instilled into the tissue space surrounding the conduit via the port, the endoscope and camera control unit and monitor optionally configured to display a 3D image on the monitor, a means for viewing a 3D image on a monitor.

The present disclosure includes a surgical kit including a monitor drape having one or more of the following features, in any combination. In particular any one or more of the following features of a monitor drape may be combined with the apparatus according to the first aspect of the present invention, or with the operating table arrangement according to the second aspect of the present invention.

An example of the surgical kit, for use with an operating table, particularly for use in endoscopic conduit harvesting comprises an operating table mount 400 configured for releasable attachment to an operating table 401, a support arm assembly 100 including a support arm 104, a radial arm 307 for connecting a monitor mounting bar 305 substantially parallel to the support arm 104, a collet clamp assembly 200 comprising a collet 210 and a collet clamp 220 sized for tightening upon the support arm 104, a monitor mounting bracket 700 that is attachable to the support arm 104 and has a flange extending from a wall of the mounting bracker 700, a monitor mount comprising a friction block 703 with screw holes for fasteners to fix the friction block 703 to the rear surface of a monitor 500, the screw holes being optionally in a VESA configuration, the friction block 703 having a bearing surface within a central bore for receiving and frictionally engaging the mounting bar 305 for the monitor mounting bracket 700, a clamping screw 701 engageable with a thread in the monitor mount that accepts the clamping screw 701 and allows its distal end to sit against one side of the flange, and a friction assembly 300 comprising a friction clamp stem 310 adapted to frictionally engage with the distal end of the support arm 104, the radial arm 307 being fixed to a friction clamp stem 310 and extending radially from the longitudinal axis 308 of the support arm 104, and wherein the mounting bar 305 is fixable to the radial arm 307 to extend spaced from and parallel to the longitudinal axis 308 of the support arm 104.

In such a surgical kit the support arm 104 may be operatively associated with a linear actuator for raising and lowering the support arm assembly 100.

The said surgical kit may include an on table monitor 500 positionable upon, and supportable by the support arm assembly 100, and a monitor drape 600 including a fenestration to pass the monitor mounting bar 305,

The monitor drape 600 may comprise a sheath with one open end 606 and one closed end 604, an optically transparent window 607 secured to a fenestration 605 cut into the sheath, and peel-off protective cover 602 over the optically transparent window 607.

An example of the monitor drape may include one or more of the following features:
a sheath with one open end and one closed end, optically transparent window cut into sheath, peel-off protective cover over optically transparent window, lateral restrictions on the width of sheath (seams) at the 'closed' end, reinforcing patch on surface of sheath opposite the optically clear window incorporating a fenestration, the sheath being made from a stretchable material so that it firmly 'clasps' a monitor slid to the closed end of the sheath between the two lateral restrictions, the sheath being made from a material that can be sterilised without losing its physical integrity or the above stretchable properties which are essential to its function.

The invention is not limited to the specific embodiments and methods described, and modifications and alternatives are possible. The shape, material and size of the various components can be modified, and additional method steps may be included, within the scope of the invention as defined by the claims.

## Claims

1. A monitor mount apparatus (10) for use in an operating theatre comprising:
a mount (400) attachable to an operating table (401),
a support arm (104) connected by a clamp assembly (200) to the mount (400), the support arm (104) extending along its longitudinal axis in a horizontal plane parallel to a plane of the operating table (401),
a monitor mounting bracket (700) connected by a friction assembly (300) to a distal end of the support arm (104) distal from the clamp assembly (200), and
a display monitor (500) adapted to display an image from an endoscope (1000) and fixedly supported by the monitor mounting bracket (700);
wherein the friction assembly (300) is adapted to permit under hand operation of a user:
tilting of the monitor mounting bracket (700) about a tilt axis parallel to the longitudinal axis (308) of the support arm (104), and
vertical movement of the monitor mounting bracket (700) relative to the support arm (104); and
wherein the friction assembly (300) prevents said tilting and vertical movement under self weight of the display monitor (500) and the monitor mounting bracket (700), and
wherein the friction assembly (300) comprises:
a friction clamp stem (310) adapted to frictionally engage with the distal end of the support arm (104),
a radial arm (307) fixed to the friction clamp stem (310) and extending radially from the longitudinal axis (308) of the support arm (104),
a mounting bar (305) fixed to the radial arm (307) and extending spaced from and parallel to the longitudinal axis (308) of the support arm (104), and
a friction clamp mechanism (701,703) adapted to frictionally engage the monitor mounting bracket (700) with the mounting bar (305);
a clamp screw (701) having a thread engaged in a threaded aperture (702) of the mounting bracket (700), and
a friction block (703) mounted resiliently to the mounting bracket (700) and engageable with a distal end of the clamp screw (701),
wherein the friction block (703) is adapted to frictionally engage the mounting bar (305) between two bearing apertures (705) of the monitor mounting bracket (700) through which the mounting bar (305) extends.

2. Apparatus according to claim 1, wherein the friction assembly (300) is further adapted to permit under hand operation of a user:
translational movement of the monitor mounting bracket (700) along the tilt axis, and
wherein the friction assembly prevents said translational movement under self weight of the display monitor (500) and the monitor mounting bracket (700).

3. Apparatus according to claim 1 or 2, wherein the support arm (104) is pivotally connected by the clamp assembly (200) to the mount, and the clamp assembly (200) is operable between an unclamped state in which the support arm (104) can rotate about a vertical axis (106) and a clamped state in which the support arm (104) is fixed or frictionally fixed relative to the mount (400) in a working position.

4. Apparatus according to any preceding claim, wherein the display monitor (500) has a visible display area with a maximum diagonal dimension of less than 410 mm and/or a maximum weight of 2.5 kg.

5. Apparatus according to claim 1, wherein the friction clamp stem (310) is a quill stem comprising:
a tubular sleeve portion (303) extending from the radial arm (307), adapted to fit slidingly within a tubular sleeve of the support arm (104) and having a wedge bearing surface (303A) at its end distal from the radial arm (307),
a threaded wedge nut (304) adapted to fit slidingly within the tubular sleeve of the support arm (104) and having a wedge bearing surface (304A) adapted to engage the wedge bearing surface (303A) of the tubular sleeve portion (303), and
a threaded screw member (301) having a hand-engageable knob portion (306) at a first end and a screw thread (311) at a second end engageable in the threaded wedge nut (304).

6. Apparatus according to claim 5, wherein the friction clamp force of the friction clamp stem (310) is adjustable by hand rotation of the knob portion (306) by an operator.

7. Apparatus according to claim 1, wherein the friction clamp force of the friction clamp mechanism (701, 703) is adjustable by hand rotation of the clamp screw (701) by an operator.

8. Apparatus according to either of claims 1 or 7, wherein the friction block (703) is adapted to permit axial rotation of the mounting bar (305) relative to the friction block (703) under hand operation of a user while the friction block (703) frictionally engages the mounting bar (305), thereby allowing tilting of the monitor mounting bracket (700) about the tilt axis.

9. Apparatus according to any one of claims 1 or 7 or 8, wherein the friction block (703) is adapted to permit axial movement of the mounting bar (305) relative to the friction block (703) under hand operation of a user while the friction block (703) frictionally engages the mounting bar (305), thereby allowing translational movement of the monitor mounting bracket (700) along the tilt axis.

10. Apparatus according to any one of claims 1 or 7 to 9, wherein the friction clamp stem (310) is adapted to permit rotation of the radial arm (307) and mounting bar (305) about the longitudinal axis of the support arm (104) under hand operation of a user while the friction clamp stem (310) frictionally engages with the distal end of the support arm (104), thereby allowing vertical movement of the monitor mounting bracket (700) relative to the support arm (104).

11. An operating table arrangement for use in an operating theatre, the arrangement comprising:
an operating table (401), and
an apparatus according to any of claims 1 to 10,
wherein the mount (400) is attached to the operating table (401),
wherein the display monitor (500) is positioned in a location suitable for viewing by an endoscopic vessel harvesting (EVH) operator (1300) using an endoscope (1000), and
wherein the display monitor (500) is adapted to display an image from the endoscope (1000).

12. A method of displaying an image from an endoscope (1000), comprising:
providing an apparatus according to any of claims 1 to 10, wherein the mount (400) is attached to an operating table,
providing an endoscope (1000),
frictionally clamping the support arm (104) to the mount (400) using the clamp assembly (200), such that the support arm (104) extending along its longitudinal axis (308) in a horizontal plane parallel to a plane of the operating table, and the display monitor is in a coarse position adjacent to a working location on or adjacent to the operating table so that an image from the endoscope (1000) is viewable on the display monitor by a user at the working location,
applying a force by hand operation to the display monitor (500) to provide fine adjustment of the display monitor position, by one or more of:
tilting of the monitor mounting bracket (700) about a tilt axis parallel to the longitudinal axis (308) of the support arm (104),
translational movement of the monitor mounting bracket along the tilt axis, and
vertical movement of the monitor mounting bracket relative to the support arm (104).

13. The method of claim 12, further including the step of grasping and rotating a rotatable adjusting sleeve to adjust a friction brake of the clamp assembly.

14. The method of claim 12 or 13, further including the following step or steps:
adjusting the friction clamp force of the friction clamp stem (310) by hand rotation of the knob portion of the threaded screw member of the friction clamp (310), and/or
adjusting the friction clamp force of the friction clamp mechanism (701, 703) by hand rotation of the head portion (711) of the clamp screw (701) of the friction clamp mechanism (701, 703).

15. The method of any of claims 12 to 14, further including one or more of the following steps:
providing fine tilt adjustment of the display monitor position, by axially rotating the mounting bar (305) relative to the friction block (703)under hand operation of a user while the friction block (703) frictionally engages the mounting bar (305), thereby allowing tilting of the monitor mounting bracket (700) about the tilt axis;
and/or
providing fine horizontal adjustment of the display monitor position, by axially moving the mounting bar (305) relative to the friction block (703) under hand operation of a user while the friction block (703) frictionally engages the mounting bar (305), thereby allowing translational movement of the monitor mounting bracket (700) along the tilt axis; and/or
providing fine vertical adjustment of the display monitor position, by rotating the radial arm (307) and mounting bar (305) about the longitudinal axis of the support arm (104) under hand operation of a user while the friction clamp stem (310) frictionally engages with the distal end of the support arm (104), thereby allowing vertical movement of the monitor mounting bracket (700) relative to the support arm (104).

## Patentansprüche

1. Eine Monitorbefestigungseinrichtungsvorrichtung (10) zur Verwendung in einem Operationssaal, die Folgendes beinhaltet:
eine Befestigungseinrichtung (400), die an einem Operationstisch (401) angebracht werden kann,
einen Tragarm (104), der durch eine Klemmanordnung (200) mit der Befestigungseinrichtung (400) verbunden ist, wobei sich der Tragarm (104) entlang seiner Längsachse in einer horizontalen Ebene parallel zu einer Ebene des Operationstisches (401) erstreckt,
einen Monitorbefestigungsbügel (700), der durch eine Reibungsanordnung (300) mit einem distalen Ende des Tragarms (104) distal entfernt von der Klemmanordnung (200) verbunden ist, und
einen Anzeigemonitor (500), der zur Anzeige eines Bildes von einem Endoskop (1000) angepasst ist und von dem Monitorbefestigungsbügel (700) fixiert getragen wird;
wobei die Reibungsanordnung (300) angepasst ist, um eine Unter-Hand-Bedienung durch einen Benutzer zu ermöglichen:
Kippen des Monitorbefestigungsbügels (700) um eine Kippachse parallel zu der Längsachse (308) des Tragarms (104) und
eine vertikale Bewegung des Monitorbefestigungsbügels (700) relativ zu dem Tragarm (104); und
wobei die Reibungsanordnung (300) das Kippen und die vertikale Bewegung unter dem Eigengewicht des Anzeigemonitors (500) und des Monitorbefestigungsbügels (700) verhindert und
wobei die Reibungsanordnung (300) Folgendes beinhaltet:
einen Reibklemmschaft (310), der angepasst ist, um mit dem distalen Ende des Tragarms (104) in Reibungseingriff zu kommen,
einen radialen Arm (307), der an dem Reibklemmschaft (310) fixiert ist und sich radial von der Längsachse (308) des Tragarms (104) erstreckt,
eine Befestigungsstange (305), die an dem radialen Arm (307) fixiert ist und sich beabstandet von und parallel zu der Längsachse (308) des Tragarms (104) erstreckt, und
einen Reibungsklemmmechanismus (701, 703), der angepasst ist, um den Monitorbefestigungsbügel (700) in Reibungseingriff mit der Befestigungsstange (305) zu bringen;
eine Klemmschraube (701) mit einem Gewinde, das mit einer Gewindeöffnung (702) des Befestigungsbügels (700) in Eingriff kommt, und
einen Reibungsblock (703), der elastisch an dem Befestigungsbügel (700) befestigt ist und mit einem distalen Ende der Klemmschraube (701) in Eingriff gebracht werden kann,
wobei der Reibungsblock (703) angepasst ist, um mit der Befestigungsstange (305) zwischen zwei Lageröffnungen (705) des Monitorbefestigungsbügels (700), durch die sich die Befestigungsstange (305) erstreckt, in Reibungseingriff zu kommen.

2. Vorrichtung gemäß Anspruch 1, wobei die Reibungsanordnung (300) ferner angepasst ist, um eine Unter-Hand-Bedienung durch einen Benutzer zu ermöglichen:
eine Translationsbewegung des Monitorbefestigungsbügels (700) entlang der Kippachse, und
wobei die Reibungsanordnung die Translationsbewegung unter dem Eigengewicht des Anzeigemonitors (500) und des Monitorbefestigungsbügels (700) verhindert.

3. Vorrichtung gemäß Anspruch 1 oder 2, wobei der Tragarm (104) durch die Klemmanordnung (200) schwenkbar mit der Befestigungseinrichtung verbunden ist und die Klemmanordnung (200) zwischen einem ungeklemmten Zustand, in dem sich der Tragarm (104) um eine vertikale Achse (106) drehen kann, und einem geklemmten Zustand, in dem der Tragarm (104) relativ zu der Befestigungseinrichtung (400) in einer Arbeitsposition fixiert oder reibschlüssig fixiert ist, bedient werden kann.

4. Vorrichtung gemäß einem der vorhergehenden Ansprüche, wobei der Anzeigemonitor (500) eine sichtbare Anzeigefläche mit einer maximalen Diagonalabmessung von weniger als 410 mm und/oder einem maximalen Gewicht von 2,5 kg aufweist.

5. Vorrichtung gemäß Anspruch 1, wobei der Reibklemmschaft (310) ein Pinolenschaft ist, der Folgendes beinhaltet:
einen sich von dem radialen Arm (307) aus erstreckenden rohrförmigen Hülsenabschnitt (303), der angepasst ist, um gleitend in eine rohrförmige Hülse des Tragarms (104) zu passen, und der an seinem von dem radialen Arm (307) entfernten Ende eine Keillagerfläche (303A) aufweist,
eine Keilmutter (304) mit Gewinde, die angepasst ist, um gleitend in die rohrförmige Hülse des Tragarms (104) zu passen, und eine Keillagerfläche (304A) aufweist, die angepasst ist, um mit der Keillagerfläche (303A) des rohrförmigen Hülsenabschnitts (303) in Eingriff zu kommen, und
ein Gewindeschraubelement (301) mit einem Knopfabschnitt (306), der von Hand in Eingriff genommen werden kann, an einem ersten Ende und einem Schraubengewinde (311) an einem zweiten Ende, das mit der Keilmutter (304) mit Gewinde in Eingriff kommen kann.

6. Vorrichtung gemäß Anspruch 5, wobei die Reibklemmkraft des Reibklemmschafts (310) durch Handdrehung des Knopfabschnittes (306) durch eine Bedienungsperson einstellbar ist.

7. Vorrichtung gemäß Anspruch 1, wobei die Reibklemmkraft des Reibklemmmechanismus (701, 703) durch Handdrehung der Klemmschraube (701) durch eine Bedienungsperson einstellbar ist.

8. Vorrichtung gemäß einem der Ansprüche 1 oder 7, wobei der Reibungsblock (703) angepasst ist, um eine axiale Drehung der Befestigungsstange (305) relativ zu dem Reibungsblock (703) durch Unter-Hand-Bedienung durch einen Benutzer zu ermöglichen, während der Reibungsblock (703) reibschlüssig mit der Befestigungsstange (305) in Eingriff steht, wodurch ein Kippen des Monitorbefestigungsbügels (700) um die Kippachse ermöglicht wird.

9. Vorrichtung gemäß einem der Ansprüche 1 oder 7 oder 8, wobei der Reibungsblock (703) angepasst ist, um eine axiale Bewegung der Befestigungsstange (305) relativ zu dem Reibungsblock (703) durch Unter-Hand-Bedienung durch einen Benutzer zu ermöglichen, während der Reibungsblock (703) reibschlüssig mit der Befestigungsstange (305) in Eingriff steht, wodurch eine Translationsbewegung des Monitorbefestigungsbügels (700) entlang der Kippachse ermöglicht wird.

10. Vorrichtung gemäß einem der Ansprüche 1 oder 7 bis 9, wobei der Reibklemmschaft (310) angepasst ist, um eine Drehung des radialen Arms (307) und der Befestigungsstange (305) um die Längsachse des Tragarms (104) durch Unter-Hand-Bedienung durch einen Benutzer zu ermöglichen, während der Reibklemmschaft (310) reibschlüssig mit dem distalen Ende des Tragarms (104) in Eingriff steht, wodurch eine vertikale Bewegung des Monitorbefestigungsbügels (700) relativ zu dem Tragarm (104) ermöglicht wird.

11. Eine Operationstischanlage zur Verwendung in einem Operationssaal, wobei die Anlage Folgendes beinhaltet:
einen Operationstisch (401), und
eine Vorrichtung gemäß einem der Ansprüche 1 bis 10,
wobei die Befestigungseinrichtung (400) an dem Operationstisch (401) angebracht ist, wobei der Anzeigemonitor (500) an einer Stelle positioniert ist, die für die Betrachtung durch eine Bedienungsperson (1300) der endoskopischen Gefäßentnahme (EVH, Endoscopic Vessel Harvesting) unter Verwendung eines Endoskops (1000) geeignet ist, und
wobei der Anzeigemonitor (500) angepasst ist, um ein Bild von dem Endoskop (1000) anzuzeigen.

12. Verfahren zum Anzeigen eines Bildes von einem Endoskop (1000), das Folgendes beinhaltet:
Bereitstellen einer Vorrichtung gemäß einem der Ansprüche 1 bis 10, wobei die Befestigungseinrichtung (400) an einem Operationstisch angebracht ist,
Bereitstellen eines Endoskops (1000),
reibschlüssiges Klemmen des Tragarms (104) an die Befestigungseinrichtung (400) unter Verwendung der Klemmanordnung (200), sodass sich der Tragarm (104) entlang seiner Längsachse (308) in einer horizontalen Ebene parallel zu einer Ebene des Operationstisches erstreckt und der Anzeigemonitor sich in einer groben Position benachbart zu dem Arbeitsort auf oder benachbart zu dem Operationstisch befindet, sodass ein Bild von dem Endoskop (1000) auf dem Anzeigemonitor durch einen Benutzer an dem Arbeitsort betrachtet werden kann,
Aufbringen einer Kraft durch Handbedienung auf den Anzeigemonitor (500), um eine Feineinstellung der Position des Anzeigemonitors bereitzustellen, durch eines oder mehrere von Folgendem:
Kippen des Monitorbefestigungsbügels (700) um eine Kippachse parallel zu der Längsachse (308) des Tragarms (104),
eine Translationsbewegung des Monitorbefestigungsbügels entlang der Kippachse, und
eine vertikale Bewegung des Monitorbefestigungsbügels relativ zu dem Tragarm (104).

13. Verfahren gemäß Anspruch 12, ferner umfassend den Schritt des Ergreifens und Drehens einer drehbaren Einstellhülse zum Einstellen einer Reibungsbremse der Klemmanordnung.

14. Verfahren gemäß Anspruch 12 oder 13, das ferner den folgenden Schritt oder die folgenden Schritte umfasst:
Einstellen der Reibklemmkraft des Reibklemmschaftes (310) durch Handdrehung des Knopfabschnitts des Gewindeschraubelements der Reibungsklemme (310), und/oder Einstellen der Reibklemmkraft des Reibungsklemmmechanismus (701, 703) durch Handdrehung des Kopfabschnittes (711) der Klemmschraube (701) des Reibungsklemmmechanismus (701, 703).

15. Verfahren gemäß einem der Ansprüche 12 bis 14, das ferner einen oder mehrere der folgenden Schritte umfasst:
Bereitstellen einer feinen Kippeinstellung der Position des Anzeigemonitors durch axiales Drehen der Befestigungsstange (305) relativ zu dem Reibungsblock (703) durch Unter-Hand-Bedienung durch einen Benutzer, während der Reibungsblock (703) reibschlüssig an der Befestigungsstange (305) in Eingriff kommt, wodurch ein Kippen des Monitorbefestigungsbügels (700) um die Kippachse ermöglicht wird;
und/oder
Bereitstellen einer feinen horizontalen Einstellung der Position des Anzeigemonitors durch axiales Bewegen der Befestigungsstange (305) relativ zu dem Reibungsblock (703) durch Unter-Hand-Bedienung durch einen Benutzer, während der Reibungsblock (703) reibschlüssig an der Befestigungsstange (305) in Eingriff kommt, wodurch eine Translationsbewegung des Monitorbefestigungsbügels (700) entlang der Kippachse ermöglicht wird; und/oder
Bereitstellen einer feinen vertikalen Einstellung der Position des Anzeigemonitors durch Drehen des radialen Arms (307) und der Befestigungsstange (305) um die Längsachse des Tragarms (104) durch Unter-Hand-Bedienung durch einen Benutzer, während der Reibklemmschaft (310) reibschlüssig mit dem distalen Ende des Tragarms (104) in Eingriff kommt, wodurch eine vertikale Bewegung des Monitorbefestigungsbügels (700) relativ zu dem Tragarm (104) ermöglicht wird.

## Revendications

1. Un appareil à support de moniteur (10) pour son utilisation dans une salle d'opération, comprenant :
un support (400) pouvant être attaché à une table d'opération (401),
un bras de soutien (104) raccordé par un ensemble de serrage (200) au support (400), le bras de soutien (104) s'étendant le long de son axe longitudinal dans un plan horizontal parallèlement à un plan de la table d'opération (401),
un étrier de montage (700) de moniteur raccordé par un ensemble à frottement (300) à une extrémité distale du bras de soutien (104) distalement par rapport à l'ensemble de serrage (200), et
un moniteur d'affichage (500) conçu pour afficher une image provenant d'un endoscope (1000) et soutenu de façon fixe par l'étrier de montage (700) de moniteur ;
où l'ensemble à frottement (300) est conçu pour permettre, sous actionnement manuel d'un utilisateur :
le fait d'incliner l'étrier de montage (700) de moniteur autour d'un axe d'inclinaison parallèlement à l'axe longitudinal (308) du bras de soutien (104), et
un déplacement vertical de l'étrier de montage (700) de moniteur relativement au bras de soutien (104) ; et
où l'ensemble à frottement (300) empêche lesdits fait d'incliner et déplacement vertical sous le propre poids du moniteur d'affichage (500) et de l'étrier de montage (700) de moniteur, et
où l'ensemble à frottement (300) comprend :
une tige de serrage à frottement (310) configurée pour se mettre en prise par frottement avec l'extrémité distale du bras de soutien (104),
un bras radial (307) fixé à la tige de serrage à frottement (310) et s'étendant radialement à partir de l'axe longitudinal (308) du bras de soutien (104),
une barre de montage (305) fixée au bras radial (307) et s'étendant espacée de et parallèlement à l'axe longitudinal (308) du bras de soutien (104), et
un mécanisme de serrage à frottement (701, 703) conçu pour mettre en prise par frottement l'étrier de montage (700) de moniteur avec la barre de montage (305) ;
une vis de serrage (701) ayant un filet en prise dans une ouverture taraudée (702) de l'étrier de montage (700), et
un bloc à frottement (703) monté de façon élastique sur le support de montage (700) et pouvant se mettre en prise avec une extrémité distale de la vis de serrage (701),
où le bloc à frottement (703) est conçu pour mettre en prise par frottement la barre de montage (305) entre deux ouvertures portantes (705) de l'étrier de montage (700) de moniteur à travers lesquelles la barre de montage (305) s'étend.

2. Appareil selon la revendication 1, où l'ensemble à frottement (300) est conçu en outre pour permettre, sous actionnement manuel d'un utilisateur :
un déplacement en translation de l'étrier de montage (700) de moniteur le long de l'axe d'inclinaison, et
où l'ensemble à frottement empêche ledit déplacement en translation sous le propre poids du moniteur d'affichage (500) et de l'étrier de montage (700) de moniteur.

3. Appareil selon la revendication 1 ou la revendication 2, où le bras de soutien (104) est raccordé de façon pivotante par l'ensemble de serrage (200) au support, et l'ensemble de serrage (200) peut être actionné entre un état desserré dans lequel le bras de soutien (104) peut tourner autour d'un axe vertical (106) et un état serré dans lequel le bras de soutien (104) est fixé ou fixé par frottement relativement au support (400) dans une position de travail.

4. Appareil selon n'importe quelle revendication précédente, où le moniteur d'affichage (500) a une zone d'affichage visible avec une dimension en diagonale maximale de moins de 410 mm et/ou un poids maximal de 2,5 kg.

5. Appareil selon la revendication 1, où la tige de serrage à frottement (310) est une tige formant fourreau comprenant :
une partie formant manchon tubulaire (303) s'étendant à partir du bras radial (307), conçue pour s'ajuster de façon à coulisser à l'intérieur d'un manchon tubulaire du bras de soutien (104) et ayant une surface portante de calage (303A) au niveau de son extrémité distale par rapport au bras radial (307),
un écrou de calage taraudé (304) conçu pour s'ajuster de façon à coulisser à l'intérieur du manchon tubulaire du bras de soutien (104) et ayant une surface portante de calage (304A) conçue pour mettre en prise la surface portante de calage (303A) de la partie formant manchon tubulaire (303), et
un organe formant vis filetée (301) ayant une partie formant bouton pouvant être mise en prise manuellement (306) au niveau d'une première extrémité et un filet de vis (311) au niveau d'une deuxième extrémité pouvant être mis en prise dans l'écrou de calage taraudé (304).

6. Appareil selon la revendication 5, où la force de serrage par frottement de la tige de serrage à frottement (310) peut être réglée par rotation manuelle de la partie formant bouton (306) par un utilisateur.

7. Appareil selon la revendication 1, où la force de serrage par frottement du mécanisme de serrage à frottement (701, 703) peut être réglée par rotation manuelle de la vis de serrage (701) par un opérateur.

8. Appareil selon soit la revendication 1, soit la revendication 7, où le bloc à frottement (703) est conçu pour permettre une rotation axiale de la barre de montage (305) relativement au bloc à frottement (703) sous actionnement manuel d'un utilisateur tandis que le bloc à frottement (703) met en prise par frottement la barre de montage (305), rendant ainsi possible le fait d'incliner l'étrier de montage (700) de moniteur autour de l'axe d'inclinaison.

9. Appareil selon n'importe laquelle des revendications 1 ou 7 ou 8, où le bloc à frottement (703) est conçu pour permettre un déplacement axial de la barre de montage (305) relativement au bloc à frottement (703) sous actionnement manuel d'un utilisateur tandis que le bloc à frottement (703) met en prise par frottement la barre de montage (305), rendant ainsi possible un déplacement en translation de l'étrier de montage (700) de moniteur le long de l'axe d'inclinaison.

10. Appareil selon n'importe laquelle des revendications 1 ou 7 à 9, où la tige de serrage à frottement (310) est conçue pour permettre une rotation du bras radial (307) et de la barre de montage (305) autour de l'axe longitudinal du bras de soutien (104) sous actionnement manuel d'un utilisateur tandis que la tige de serrage à frottement (310) se met en prise par frottement avec l'extrémité distale du bras de soutien (104), rendant ainsi possible un déplacement vertical de l'étrier de montage (700) de moniteur relativement au bras de soutien (104).

11. Un agencement de table d'opération pour son utilisation dans une salle d'opération, l'agencement comprenant :
un table d'opération (401), et
un appareil selon n'importe lesquelles des revendications 1 à 10,
où le support (400) est attaché à la table d'opération (401),
où le moniteur d'affichage (500) est positionné dans un emplacement convenant pour être visualisé par un opérateur de prélèvement endoscopique de vaisseaux (PEV) (1300) utilisant un endoscope (1000), et
où le moniteur d'affichage (500) est conçu pour afficher une image provenant de l'endoscope (1000).

12. Un procédé d'affichage d'une image provenant d'un endoscope (1000), comprenant :
le fait de fournir un appareil selon n'importe lesquelles des revendications 1 à 10, où le support (400) est attaché à une table d'opération,
le fait de fournir un endoscope (1000),
le fait de serrer par frottement le bras de soutien (104) sur le support (400) en utilisant l'ensemble de serrage (200), de telle sorte que le bras de soutien (104) s'étend le long de son axe longitudinal (308) dans un plan horizontal parallèlement à un plan de la table d'opération, et le moniteur d'affichage est dans une position approximative adjacente à un emplacement de travail sur ou adjacente à la table d'opération de telle sorte qu'une image provenant de l'endoscope (1000) peut être visualisée sur le moniteur d'affichage par un utilisateur au niveau de l'emplacement de travail,
le fait d'appliquer une force par actionnement manuel sur l'écran d'affichage (500) afin de fournir un réglage précis de la position du moniteur d'affichage, par un ou plusieurs éléments parmi :
le fait d'incliner l'étrier de montage (700) de moniteur autour d'un axe d'inclinaison parallèlement à l'axe longitudinal (308) du bras de soutien (104),
un déplacement en translation de l'étrier de montage de moniteur le long de l'axe d'inclinaison, et
un déplacement vertical de l'étrier de montage de moniteur relativement au bras de soutien (104).

13. Le procédé de la revendication 12, incluant en outre l'étape consistant à saisir et faire tourner un manchon de réglage rotatif afin de régler un frein à frottement de l'ensemble de serrage.

14. Le procédé de la revendication 12 ou de la revendication 13, incluant en outre l'étape ou les étapes suivantes :
le fait de régler la force de serrage par frottement de la tige de serrage à frottement (310) par rotation manuelle de la partie formant bouton de l'organe formant vis filetée du serrage à frottement (310), et/ou
le fait de régler la force de serrage par frottement du mécanisme de serrage à frottement (701, 703) par rotation manuelle de la partie formant tête (711) de la vis de serrage (701) du mécanisme de serrage à frottement (701, 703).

15. Le procédé de n'importe lesquelles des revendications 12 à 14, incluant en outre une ou plusieurs des étapes suivantes :
le fait de fournir un réglage d'inclinaison précis de la position du moniteur d'affichage, en faisant tourner axialement la barre de montage (305) relativement au bloc à frottement (703) sous actionnement manuel d'un utilisateur tandis que le bloc à frottement (703) met en prise par frottement la barre de montage (305), rendant ainsi possible le fait d'incliner l'étrier de montage (700) de moniteur autour de l'axe d'inclinaison ;
et/ou
le fait de fournir un réglage horizontal précis de la position du moniteur d'affichage, en déplaçant axialement la barre de montage (305) relativement au bloc à frottement (703) sous actionnement manuel d'un utilisateur tandis que le bloc à frottement (703) met en prise par frottement la barre de montage (305), rendant ainsi possible un déplacement en translation de l'étrier de montage (700) de moniteur le long de l'axe d'inclinaison ;
et/ou
le fait de fournir un réglage vertical précis de la position du moniteur d'affichage, en faisant tourner le bras radial (307) et la barre de montage (305) autour de l'axe longitudinal du bras de soutien (104) sous actionnement manuel d'un utilisateur tandis que la tige de serrage à frottement (310) se met en prise par frottement avec l'extrémité distale du bras de soutien (104), rendant ainsi possible un déplacement vertical de l'étrier de montage (700) de moniteur relativement au bras de soutien (104).
